# EUROPEAN PATENT SPECIFICATION

(11) **EP 1 545 607 B1**
(45) Date of publication and mention of the grant of the patent: **19.11.2008**
(21) Application number: 03740967.9
(22) Date of filing: 02.06.2003
(51) Int. Cl.: A61K 39/395, A61K 39/39

(54) **ALLERGY VACCINES**
IMPFSTOFFE GEGEN ALLERGIEN
VACCINS ANTI-ALLERGIQUES

(30) Priority: 05.09.2002 US 408648 P
(43) Date of publication of application: 29.06.2005
(73) Proprietor: Resistentia Pharmaceuticals AB, 751 08 Uppsala (SE)
(72) Inventor: HELLMAN, Lars, T., 756 72 Uppsala (SE); PERSSON, Stephen, 757 57 Uppsala (SE); JANSSON, Asa, 126 52 Hagersten (SE)
(74) Representative: Bassil, Nicholas Charles
(86) International application number: PCT/IB2003/003075
(87) International publication number: WO 2004/022094

(56) References cited:
- EP-A- 1 195 161
- WO-A-00/25722
- WO-A-93/05810
- DATABASE GSP [Online] Accession no. AAB06205; 22 November 2000 (2000-11-22) XP002262968 & WO 00 25722 A 11 May 2000 (2000-05-11)
- DATABASE GSP [Online] Accession no. AAB06206; 22 November 2000 (2000-11-22) XP002262969 & WO 00 25722 A 11 May 2000 (2000-05-11)
- VERNERSSON MOLLY ET AL: "Generation of therapeutic antibody responses against IgE through vaccination" FASEB JOURNAL, vol. 16, no. 8, June 2002 (2002-06), pages 875-877, XP002262966 ISSN: 0892-6638
- BAYLOR N W ET AL: "Aluminum salts in vaccines-US perspective" VACCINE, BUTTERWORTH SCIENTIFIC. GUILDFORD, GB, vol. 20, 31 May 2002 (2002-05-31), pages S18-S23, XP004361369 ISSN: 0264-410X
- AUCOUTURIER JEROME ET AL: "Montanide ISA 720 and 51: a new generation of water in oil emulsions as adjuvants for human vaccines." EXPERT REVIEW OF VACCINES. ENGLAND JUN 2002, vol. 1, no. 1, June 2002 (2002-06), pages 111-118, XP002262967 ISSN: 1476-0584

## Description

### BACKGROUND

### 1. Technical Field

The invention relates to methods and materials involved in the use of vaccines containing a polypeptide (e.g., a chimeric IgE polypeptide) and an adjuvant. Such vaccines can be used to elicit an anti-self response (e.g., an anti-self IgE response).

### 2. Background Information

During the past few decades several diseases caused by malfunctions of the immune system have become the major challenges of modern day medicine. Two such areas are the allergic and autoimmune diseases. Allergies have become almost epidemic during the past 20-30 years. Estimations range from 20-30 percent of the total population being affected. Atopic allergies, or IgE mediated allergies, are the dominating form.

Common types of atopic allergies include hay fever, fur allergies, dust mite allergies, insect venom allergies, extrinsic asthma, and many types of food allergies. An interesting question is whether vaccines can be developed against these types of diseases. Hyposensitization therapy has been used to treat allergies since the beginning of the twentieth century (Noon, Lancet, 1:1572 (1911); and Freeman, Lancet, 1:1178 (1914)). This is an allergen-dependent treatment strategy, which involves the use of allergen extracts to treat patients by injection. Hyposensitization therapy has, however, been questioned due to often low efficacy and sometimes severe side effects. In addition, different extracts must be used for each individual form of allergy. New strategies to treat allergies thus are presently being evaluated.

Vaccines are typically administered with an adjuvant such as alum. Alum, however, is a relatively weak potentiator of cell-mediated immune responses (Krishnan et al., Infect. Immun., 68:54-63 (2000) and Gupta et al., Adjuvant properties of aluminum and calcium compounds, p. 229-248. In M. F. Powell and M. J. Newman (ed.), Vaccine design: the subunit and adjuvant approach. Plenum Press, New York, NY (1995)). In addition, aluminum hydroxide has been reported to attract eosinophils to the site of injection and increase the levels of antigen-specific and total IgE antibodies that may promote IgE-mediated allergic reactions (Baylor et al., Vaccine, 20:S18-S23 (2002); Walls, Proc. Soc. Exp. Biol. Med., 156:431-435 (1977); and Nagel et al., J. Immunol., 118:334-341 (1977)). WO-A-0025722 refers to vaccination against allergy by reducing the effect of chimeric JgE antibodies. No aluminium compound is added to Ig Epolypeptides of WO-A-0025722.

### SUMMARY

The invention provides materials and methods related to vaccines against self polypeptides. For example, the invention provides compositions containing a polypeptide (e.g., a chimeric IgE polypeptide) and an adjuvant as defined in the present claims. The polypeptide typically contains self and non-self components, which can result in both anti-self and anti non-self immune responses when administered to a mammal. For example, when administered to a mammal, the chimeric IgE polypeptides provided herein can reduce the IgE antibody effects of IgE-related diseases such as asthma, allergies, and eczema. The adjuvant typically is selected to give a relatively high anti-self response, as compared to compositions containing other adjuvants.

The invention is based on the discovery that chimeric IgE polypeptides in combination with an adjuvant can be used to reduce the level of detectable free IgE antibodies in a mammal. For example, administration of chimeric IgE polypeptides in combination with aluminum compounds unexpectedly resulted in a reduction in the levels of detectable free IgE antibodies despite previous reports that aluminum compounds increase total IgE levels.

In general, one aspect of the invention features a composition containing a polypeptide (e.g., an ORO polypeptide or an OSO polypeptide) and alum, wherein the polypeptide contains a self IgE polypeptide sequence, and wherein administration of the composition to a mammal produces an anti-self IgE antibody response with a titer dilution₅₀ value greater than 100. The composition can contain between about ten micrograms and about one gram of the polypeptide. The composition can contain about 280 micrograms of the polypeptide. The composition can contain between about ten microliters and about one milliliter of alum, The composition can contain about 50 microliters of alum. The titer dilution₅₀ value can be greater than 150, greater than 200, or greater than 400.

Another embodiment of the invention features a composition containing alum and about 280 micrograms of a polypeptide (e.g., an ORO polypeptide or an OSO polypeptide).

In another aspect, the invention features a composition for inducing an anti-self IgE antibody response in a mammal, wherein the composition is for administration to the mammal a composition under conditions wherein the mammal produces an anti-self IgE antibody response with a titer dilution₅₀ value greater than 100, wherein the composition contains a polypeptide and alum, and wherein the polypeptide contains a self polypeptide sequence from an IgE polypeptide.

Another embodiment of the invention features a composition containing a polypeptide and an aluminum compound as defined in the present claims, wherein the polypeptide contains a self IgE polypeptide sequence, and wherein administration of the composition to a mammal reduces the level of detectable free IgE in the mammal. The polypeptide can be a chimeric IgE polypeptide. The polypeptide can contain a sequence set forth in SEQ ID NO:3, SEQ ID NO:6, SEQ ID NO:10, SEQ ID NO:12, SEQ ID NO:14, SEQ ID NO:16, SEQ ID NO:18, or SEQ ID NO:21. The composition can contain between about ten micrograms and about one gram of the polypeptide. The composition can contain about 280 micrograms of the polypeptide. The aluminum compound can be an aluminum hydrogel compound. The aluminum compound can be alum. The composition can contain between about ten microliters and about one milliliter of the alum. The composition can contain about 50 microliters of the alum. The reduction can be at least about a 10 percent reduction (e.g., at least about a 20, 30, 40, 50, 60, 70, 80, 90, or 95 percent reduction). The reduction can be a reduction from about 10 percent to about 95 percent (e.g., from about 20 percent to about 95 percent, from about 25 percent to about 95 percent, from about 50 percent to about 95 percent, from about 75 percent to about 95 percent, from about 85 percent to about 95 percent, from about 25 percent to about 80 percent, or from about 50 percent to about 80 percent). The reduction can be detectable in an ELISA. An IgE receptor polypeptide sequence can be used in the ELISA. The administration of the composition to the mammal can produce an anti self IgE antibody response with a titer dilution₅₀ value greater than 100 (e.g., greater than 200, 300, 400, 500, 600, 700, 800, 900, 1000, 1100, 1200, 1300, 1400, or 1500).

Another embodiment of the invention features a composition containing an aluminum compound and about 30 to 300 micrograms of a chimeric IgE polypeptide.

Another embodiment of the invention features a composition for inducing an anti self IgE antibody response in a mammal, wherein administration to the mammal reduces the level of detectable free IgE in the mammal, wherein the composition contains a polypeptide and an aluminum compound, and wherein the polypeptide contains a self polypeptide sequence. The polypeptide can contain an amino acid sequence set forth in SEQ ID NO:3, SEQ ID NO:6, SEQ ID NO:10, SEQ ID NO:12, SEQ ID NO:14, SEQ ID NO:16, SEQ ID NO:18, or SEQ ID NO:21.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF DRAWINGS

Figure 1 is a diagram of the nucleic acid vector designated pRES-ORO.
Figure 2 is a nucleic acid sequence listing of the pRES-ORO vector (SEQ ID NO:1).
Figure 3 is a nucleic acid sequence listing of an insert sequence that encodes an ORO polypeptide (SEQ ID NO:2). The ORO polypeptide contains an opossum CH2 IgE domain followed by a rat CH3 IgE domain followed by an opossum CH4 IgE domain.
Figure 4 is an amino acid sequence listing of an ORO polypeptide (SEQ ID NO:3).
Figure 5 is a diagram of the nucleic acid vector designated pRES-OSO.
Figure 6 is a nucleic acid sequence listing of the pRES-OSO vector (SEQ ID NO:4).
Figure 7 is a nucleic acid sequence listing of an insert sequence that encodes an OSO polypeptide (SEQ ID NO:5). The OSO polypeptide contains an opossum CH2 IgE domain followed by a human CH3 IgE domain followed by an opossum CH4 IgE domain.
Figure 8 is an amino acid sequence listing of an OSO polypeptide (SEQ ID NO:6).
Figure 9 is a nucleic acid sequence listing of an insert sequence that encodes an ORORO polypeptide (SEQ ID NO:7). The ORORO polypeptide contains an opossum CH2 IgE domain followed by a rat CH3 IgE domain followed by an opossum CH2 IgE domain followed by a rat CH3 IgE domain followed by an opossum CH4 IgE domain.
Figure 10 is an amino acid sequence listing of an ORORO polypeptide (SEQ ID NO:8).
Figure 11 is a nucleic acid sequence listing of an insert sequence that encodes a modOSOSO-H polypeptide (SEQ ID NO:9). The modOSOSO-H polypeptide contains an opossum CH2 IgE domain followed by a human CH3 IgE domain followed by an opossum CH2 IgE domain followed by a human CH3 IgE domain followed by an opossum CH4 IgE domain. The modOSOSO-H polypeptide also contains point mutations in the human CH3 domains that abolish mast cell receptor binding and a C-terminal polyhistidine tag.
Figure 12 is an amino acid sequence listing of a modOSOSO-H polypeptide (SEQ ID NO:10).
Figure 13 is a nucleic acid sequence listing of an insert sequence that encodes a modOSOSO polypeptide (SEQ ID NO:11). The modOSOSO polypeptide contains an opossum CH2 IgE domain followed by a human CH3 IgE domain followed by an opossum CH2 IgE domain followed by a human CH3 IgE domain followed by an opossum CH4 IgE domain. The modOSOSO polypeptide also contains point mutations in the human CH3 domains that abolish mast cell receptor binding.
Figure 14 is an amino acid sequence listing of a modOSOSO polypeptide (SEQ ID NO:12).
Figure 15 is a nucleic acid sequence listing of an insert sequence that encodes an OSO-H polypeptide (SEQ ID NO:13). The OSO-H polypeptide contains an opossum CH2 IgE domain followed by a human CH3 IgE domain followed by an opossum CH4 IgE domain. The OSO-H polypeptide also contains a C-terminal polyhistidine tag.
Figure 16 is an amino acid sequence listing of an OSO-H polypeptide (SEQ ID NO:14).
Figure 17 is a nucleic acid sequence listing of an insert sequence that encodes an OSOSO polypeptide (SEQ ID NO:15). The OSOSO polypeptide contains an opossum CH2 IgE domain followed by a human CH3 IgE domain followed by an opossum CH2 IgE domain followed by a human CH3 IgE domain followed by an opossum CH4 IgE domain.
Figure 18 is an amino acid sequence listing of an OSOSO polypeptide (SEQ ID NO:16).
Figure 19 is a nucleic acid sequence listing of an insert sequence that encodes an OSOSO-H polypeptide (SEQ ID NO:17). The OSOSO-H polypeptide contains an opossum CH2 IgE domain followed by a human CH3 IgE domain followed by an opossum CH2 IgE domain followed by a human CH3 IgE domain followed by an opossum CH4 IgE domain. The OSOSO-H polypeptide also contains a C-terminal polyhistidine tag.
Figure 20 is an amino acid sequence listing of an OSOSO-H polypeptide (SEQ ID NO:18).

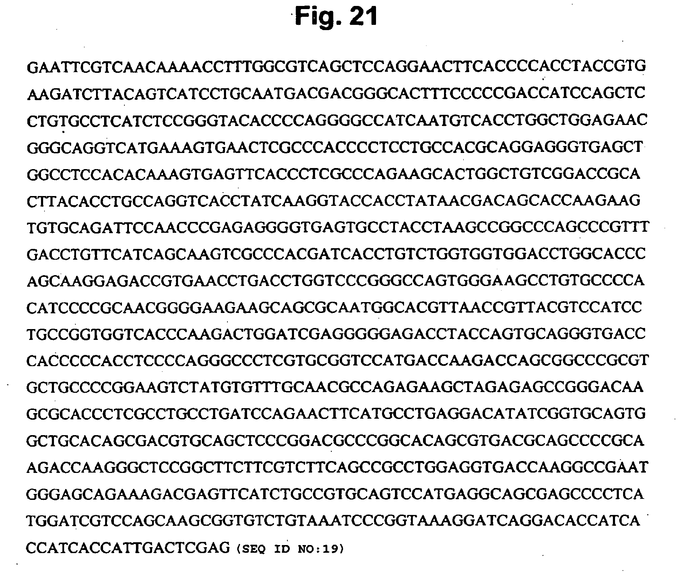

Figure 21 is a nucleic acid sequence listing of an insert sequence that encodes a CCC-H polypeptide (SEQ ID NO:19). The CCC-H polypeptide contains a monkey CH2 IgE domain followed by a monkey CH3 IgE domain followed by a monkey CH4 IgE domain followed by a polyhistidine tag.
Figure 22 is a nucleic acid sequence listing of an insert sequence that encodes a H-OCO-H polypeptide (SEQ ID NO:20). The H-OCO-H polypeptide contains an opossum CH2 IgE domain followed by a monkey CH3 IgE domain followed by an opossum CH4 IgE domain. The H-OCO-H polypeptide also contains N- and C-terminal polyhistidine tags.
Figure 23 is an amino acid sequence listing of an H-OCO-H polypeptide (SEQ ID NO:21).
Figure 24 is a nucleic acid sequence listing of an insert sequence that encodes a H-OCOCO-H polypeptide (SEQ ID NO:22). The H-OCOCO-H polypeptide contains an opossum CH2 IgE domain followed by a monkey CH3 IgE domain followed by an opossum CH2 IgE domain followed by a monkey CH3 IgE domain followed by an opossum CH4 IgE domain. The H-OCOCO-H polypeptide also contains N- and C-terminal polyhistidine tags.
Figure 25 is a schematic of an immune response.
Figure 26 is a schematic of an IgE molecule.
Figure 27 is a schematic of a vaccine having human and opossum IgE sequences.
Figure 28 is a schematic of IgE clearance.
Figure 29A is a schematic of an H-ORO DNA construct labeling the positions of the rat and opossum IgE coding sequences. Figure 29B is a schematic showing the structure of a recombinant H-ORO polypeptide.
Figure 30A is a bar graph showing relative anti-rat IgE antibody titers (anti-self IgE) in rats vaccinated with H-ORO mixed with Freund's adjuvant, alum, or ISCOM.
Figure 30B is a bar graph showing relative anti-opossum antibody titers (anti non-self) in the same rats.
Figure 31A is a bar graph showing relative anti-rat IgE antibody titers in rats vaccinated with H-ORO mixed with Freund's adjuvant, MONTANIDE^{®} ISA 51 (MN51), or MONTANIDE^{®} ISA 720 (MN720). Figure 31B is a bar graph showing relative anti-opossum antibody titers in the same rats.
Figure 32A is a bar graph showing relative anti-rat IgE antibody titers in rats vaccinated with H-ORO mixed with MN51, with or without the addition of muramyldipeptide (MDP), monophosphoryl lipid A (MPL), and/or a formyl-methionine containing tripeptide (FM). Figure 32B is a bar graph showing relative anti-opossum antibody titers in the same rats.
Figure 33A is a bar graph showing relative anti-rat IgE antibody titers in rats vaccinated with H-ORO mixed with MN720, with or without the addition of muramyldipeptide (MDP) and/or monophosphoryl lipid A (MPL). Figure 33B is a bar graph showing relative anti-opossum antibody titers in the same rats.
Figure 34 is a line graph showing free IgE levels in sera from rats immunized with either vehicle mixed with alum or H-ORO mixed with alum.
Figure 35A is line graph showing the titer dilution curve for rat anti-IgE antibodies in serum samples from rats immunized with H-ORO mixed with MN51.
Figure 35B is a line graph showing the titer dilution curve for rat anti-IgE antibodies in serum samples from rats immunized with H-ORO mixed with alum. The broken lines represent the 95 % confidence interval of the anti-IgE response (n = 9-10).
Figure 36 is a line graph showing free IgE levels in sera from rats immunized with vehicle or ORO-H mixed with Montanide ISA 51.
Figure 37 is a line graph showing free IgE levels in sera from rats immunized with vehicle or ORORO-H mixed with Montanide ISA 51.
Figure 38A-C show the outline of a study design (A), anti-IgE titers (B), and free circulating IgE levels (C) in sera from rats immunized with vehicle, vehicle mixed with MN51, or increasing amounts of H-ORO mixed with MN51.
Figure 39 is a table listing a rat vaccination protocol for a highly purified (> 98 % pure) non-histidine tagged ORO polypeptide.
Figure 40 is a graph plotting the amount of rat IgE (ng/mL) measured in rats receiving the indicated treatment.
Figure 41 is a graph plotting the percent reduction of free circulating IgE measured in rats receiving the indicated treatment.
Figure 42 is a schematic of a monkey vaccination protocol.
Figure 43 is a schematic of an ELISA used to detect monkey anti-IgE antibodies.
Figure 44 is a line graph showing the titer dilution₅₀ values in serum samples from cynomolgus monkeys immunized with vehicle mixed with MN51, H-OCO-H mixed with MN51, or H-OCOCO-H mixed with MN5.
Figure 45 is a bar graph plotting the platelet counts for the indicated time points.
Figure 46 is a listing of the haematological measurements that were found to be normal.
Figure 47 is a schematic protocol of a monkey vaccination protocol using Alhydrogel^{™} as adjuvant.
Figure 48 is a graph plotting the titer of monkey anti-IgE antibodies for the indicated time points using different doses of H-OCO-H mixed with Alhydrogel^{™}.

### DETAILED DESCRIPTION

The invention provides methods and materials related to vaccines against self polypeptides. For example, the invention provides compositions containing a polypeptide and an adjuvant. The polypeptide typically contains self and non-self components, which can result in both anti self and anti non-self immune responses when administered to a mammal. The adjuvant typically is selected to give a relatively high anti-self response, as compared to compositions containing other adjuvants.

The term "polypeptide" as used herein refers to a chain of amino acids, regardless of length or posttranslational modification (e.g., phosphorylation or glycosylation). For example, in some embodiments, the polypeptide can be unmodified such that it lacks modifications such as phosphorylation and glycosylation. The polypeptide can contain part or all of a single naturally-occurring polypeptide, or can be a chimeric polypeptide containing amino acid sequences from two or more naturally-occurring polypeptides. An "adjuvant" is an immunological compound that can enhance an immune response against a particular antigen such as a polypeptide. Typically, the compositions of the invention are administered to a mammal such that the mammal produces antibodies against the polypeptide component of the administered composition. The mammal can be a mouse, rat, dog, cat, horse, cow, or a primate such as a human or a non-human primate (e.g., a cynomolgus monkey).

In some embodiments, the compositions of the invention can elicit an anti-self polypeptide antibody response in a mammal. For example, a polypeptide can contain one or more self polypeptide segments (e.g., a self polypeptide sequence) with or without one or more non-self polypeptide segments (e.g., a non-self polypeptide sequence). The term "self" as used herein with reference to a polypeptide sequence and a particular mammal refers to a sequence that is seen as self from the prospective of that mammal's immune system. Typically, a self polypeptide segment is an amino acid sequence that is identical or similar to a sequence from a polypeptide that is native to the species of mammal to which the composition is to be administered. The term "non-self" as used herein with reference to a polypeptide sequence and a particular mammal refers to a sequence that is seen as foreign from the prospective of that mammal's immune system. Typically, a non-self polypeptide segment is an amino acid sequence that is not native to the species of mammal to which the composition is to be administered. A polypeptide can be, for example, an ORO polypeptide that contains sequences from the rat and opossum IgE molecules and can be administered to a rat as described herein.

The polypeptides provided herein can contain more than one copy of the self segment (e.g., an ORORO polypeptide that contains two copies of a segment from the rat IgE amino acid sequence). Segments from polypeptides of any type of mammal (e.g., mouse, rat, dog, cat, horse, cow, non-human primate such as cynomolgus monkey, or human) can be included in the polypeptides provided herein. For example, any of the polypeptides described in PCT Application Serial No. PCT/SE99/01896 can be used. Alternatively, the polypeptides can contain a tag (e.g., a His tag, a myc tag, or a FLAG^{®} tag). Such tags typically are positioned at the amino terminus or the carboxyl terminus of the polypeptide, but can be positioned anywhere within the polypeptide. These tags can serve as a non-self component while aiding in the detection and/or purification of the polypeptides.

The self segment or segments, as well as the non-self segment or segments, can have any length, and typically are at least 5 amino acids in length (e.g., at least about 5, 10, 20, 30, 40, S0, 60, 65, 70, 75, 80, 85, 90, 95, 100, 105, 110, 115, 120, 125, 150, 175, 200, 500, 750, 1000, 2000, 3000, 4000, 5000, or more amino acids in length). For example, the self segment or segments, as well as the non-self segment or segments, can have a length ranging from about 20, 30, 40, 50, 60, 70, or 80 amino acids to about 90, 100, 110, 120, 130, 140, 150, 200, 250, or 500 amino acids. Typically, the self segment (or segments) of a polypeptide has an amino acid sequence that is at least 80 (e.g., 85, 90, 95, or 99) percent identical to the amino acid sequence of the polypeptide that is native to the mammal to which the composition will be administered. For example, when vaccinating a human, a self IgE segment of a chimeric IgE polypeptide can be about 110 amino acids in length with about 95 percent identity to human IgE sequences over that 110 amino acid length.

A length and percent identity over that length for any nucleic acid or amino acid sequence is determined as follows. First, a nucleic acid or amino acid sequence is compared to the identified nucleic acid or amino acid sequence using the BLAST 2 Sequences (B12seq) program from the stand-alone version of BLASTZ containing BLASTN version 2.0.14 and BLASTP version 2.0.14. This stand-alone version of BLASTZ can be obtained at Fish & Richardson's web site (www.fr.com/blast; World Wide Web at "fr" dot "com" slash "blast") or the U.S. government's National Center for Biotechnology Information web site (www.ncbi.nlm.nih.gov/blast/executables; World Wide Web at "ncbi" dot "nlm" dot "nih" dot "gov" slash "blast" slash "executables"). Instructions explaining how to use the B12seq program can be found in the readme file accompanying BLASTZ.

B12seq performs a comparison between two sequences using either the BLASTN or BLASTP algorithm. BLASTN is used to compare nucleic acid sequences, while BLASTP is used to compare amino acid sequences. To compare two nucleic acid sequences, the options are set as follows: -i is set to a file containing the first nucleic acid sequence to be compared (e.g., C:\seq1.txt); -j is set to a file containing the second nucleic acid sequence to be compared (e.g., C:\seq2.txt); -p is set to blastn; -o is set to any desired file name (e.g., C:\output.txt); -q is set to -1; -r is set to 2; and all other options are left at their default setting. For example, the following command can be used to generate an output file containing a comparison between two sequences: C:\B12seq -i c:\seq1.txt -j c:\seq2.txt -p blastn -o c:\output.txt -q -1 -r 2. To compare two amino acid sequences, the options of B12seq are set as follows: -i is set to a file containing the first amino acid sequence to be compared (e.g., C:\seq1.txt); -j is set to a file containing the second amino acid sequence to be compared (e.g., C:\seq2.txt); -p is set to blastp; -o is set to any desired file name (e.g., C:\output.txt); and all other options are left at their default setting. For example, the following command can be used to generate an output file containing a comparison between two amino acid sequences: C:\B12seq -i c:\seq1.txt -j c:\seq2.txt -p blastp -o c:\output.txt. If the target sequence shares homology with any portion of the identified sequence, then the designated output file will present those regions of homology as aligned sequences. If the target sequence does not share homology with any portion of the identified sequence, then the designated output file will not present aligned sequences. Once aligned, a length is determined by counting the number of consecutive nucleotides or amino acid residues from the target sequence presented in alignment with sequence from the identified sequence starting with any matched position and ending with any other matched position. A matched position is any position where an identical nucleotide or amino acid residue is presented in both the target and identified sequence. Gaps presented in the target sequence are not counted since gaps are not nucleotides or amino acid residues. Likewise, gaps presented in the identified sequence are riot counted since target sequence nucleotides or amino acid residues are counted, not nucleotides or amino acid residues from the identified sequence.

The percent identity over a determined length is determined by counting the number of matched positions over that length and dividing that number by the length followed by multiplying the resulting value by 100. For example, if (1) a 1000 amino acid target sequence is compared to a 200 amino acid test sequence, (2) the B12seq program presents 200 amino acids from the target sequence aligned with a region of the test sequence where the first and last nucleotides of that 200 nucleotide region are matches, and (3) the number of matches over those 200 aligned nucleotides is 180, then the 1000 nucleotide target sequence contains a length of 200 and a percent identity over that length of 90 (i.e., 180 / 200 * 100 = 90).

It will be appreciated that a single nucleic acid or amino acid target sequence that aligns with an identified sequence can have many different lengths with each length having its own percent identity. For example, a target sequence containing a 20 nucleotide region that aligns with an identified sequence as follows has many different lengths including those listed in Table 1.

1                  20
Target Sequence:      AGGTCGTGTACTGTCAGTCA (SEQ ID NO:23)
                      | || ||| |||| |||| |
Identified Sequence:  ACGTGGTGAACTGCCAGTGA (SEQ ID NO:24)

**Table I.**

| Starting Position | Ending Position | Length | Matched Positions | Percent Identity |
|---|---|---|---|---|
| 1 | 20 | 20 | 15 | 75.0 |
| 1 | 18 | 18 | 14 | 77.8 |
| 1 | 15 | 15 | 11 | 73.3 |
| 6 | 20 | 15 | 12 | 80.0 |
| 6 | 17 | 12 | 10 | 83.3 |
| 6 | 15 | 10 | 8 | 80.0 |
| 8 | 20 | 13 | 10 | 76.9 |
| 8 | 16 | 9 | 7 | 77.8 |

It is noted that the percent identity value is rounded to the nearest tenth. For example, 78.11, 78.12, 78.13, and 78.14 is rounded down to 78.1, while 78.15, 78.16, 78.17, 78.18, and 78.19 is rounded up to 78.2. It is also noted that the length value will always be an integer.

Any method can be used to obtain a polypeptide. For example, molecular cloning techniques can be used to prepare a nucleic acid construct encoding a polypeptide containing self and non-self segments (e.g., ORO). Such a construct can be expressed in an organism such as *E. coli* or *S. cerevisiae*, or in a cell line, for example, and then can be purified from cellular extracts or from culture supernatants. Alternatively, a polypeptide can be chemically synthesized.

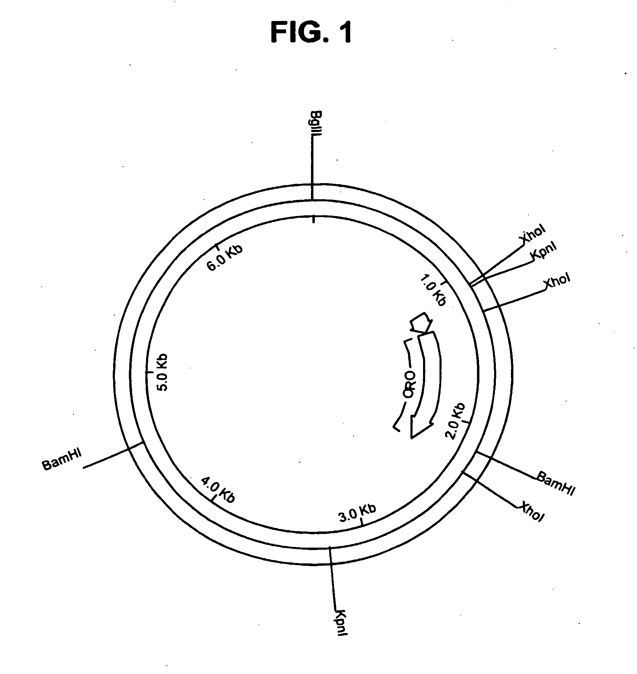

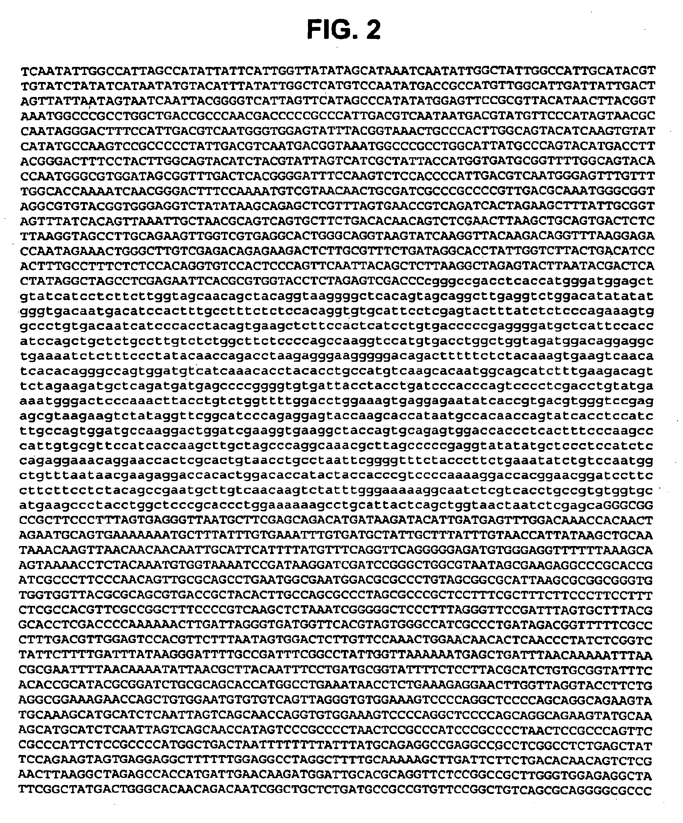

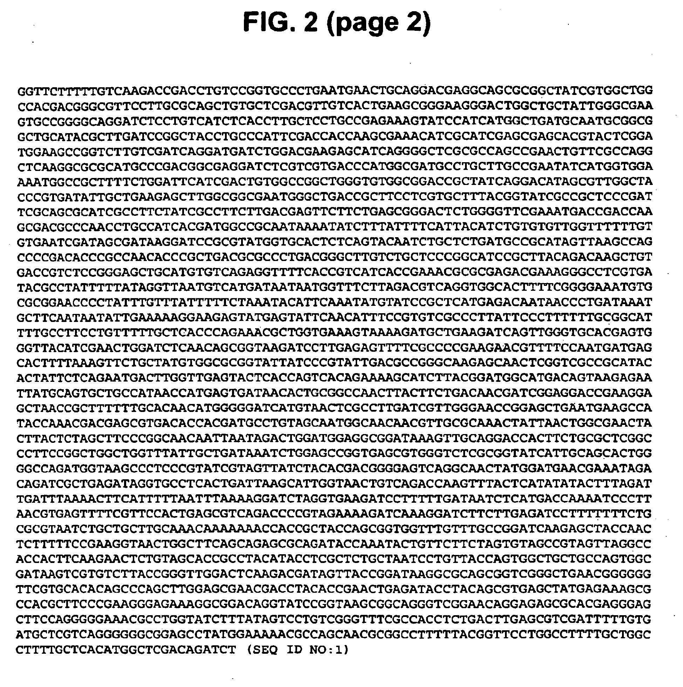

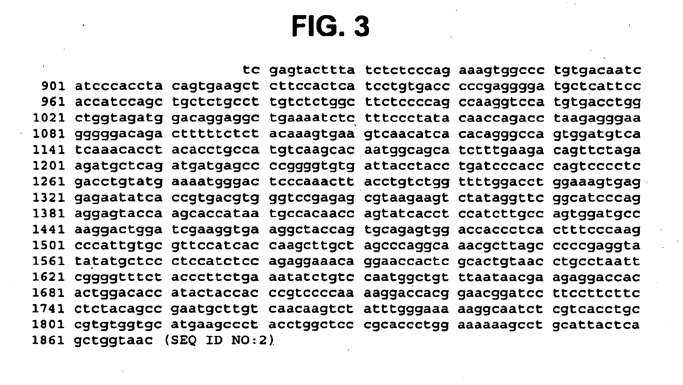

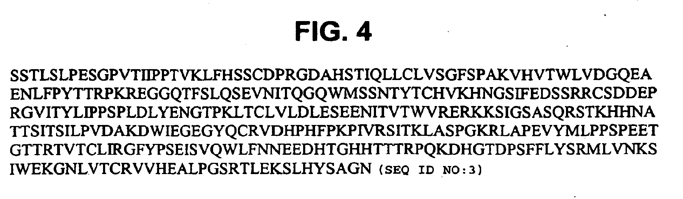

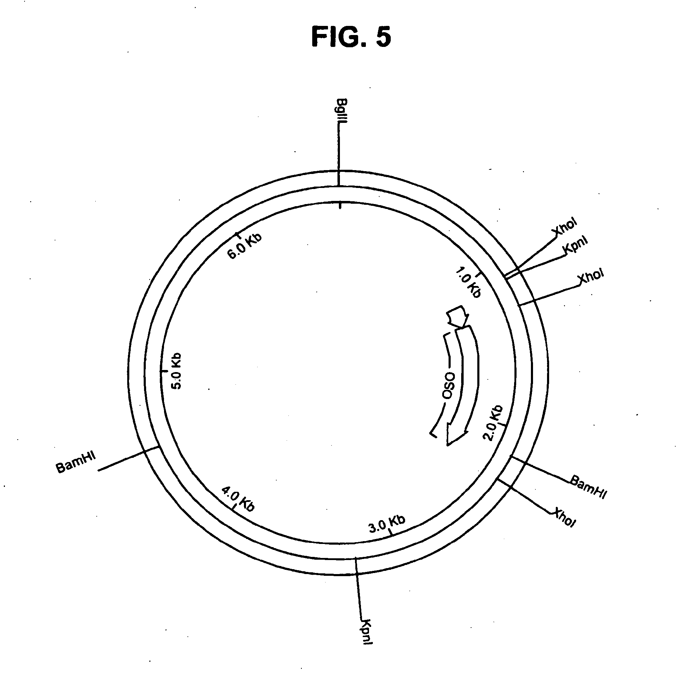

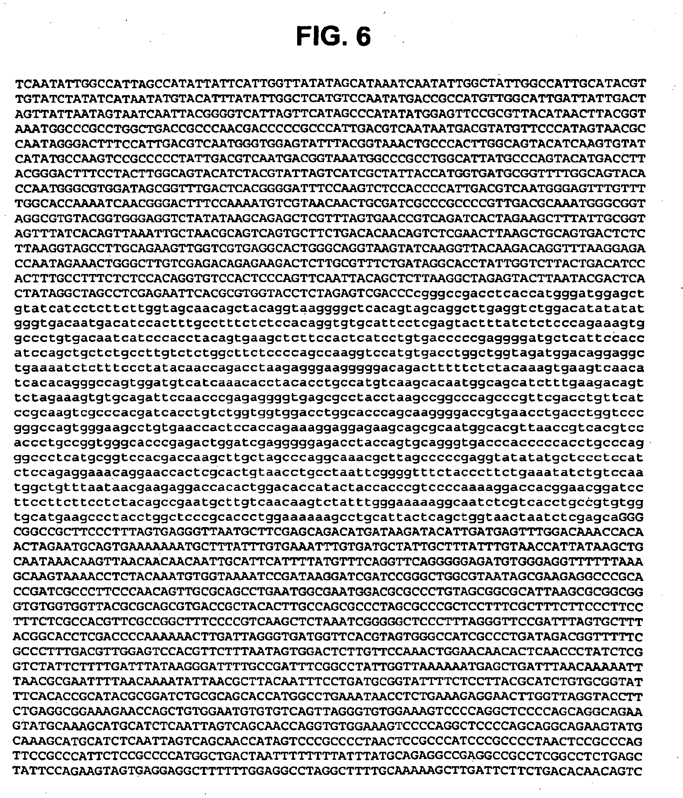

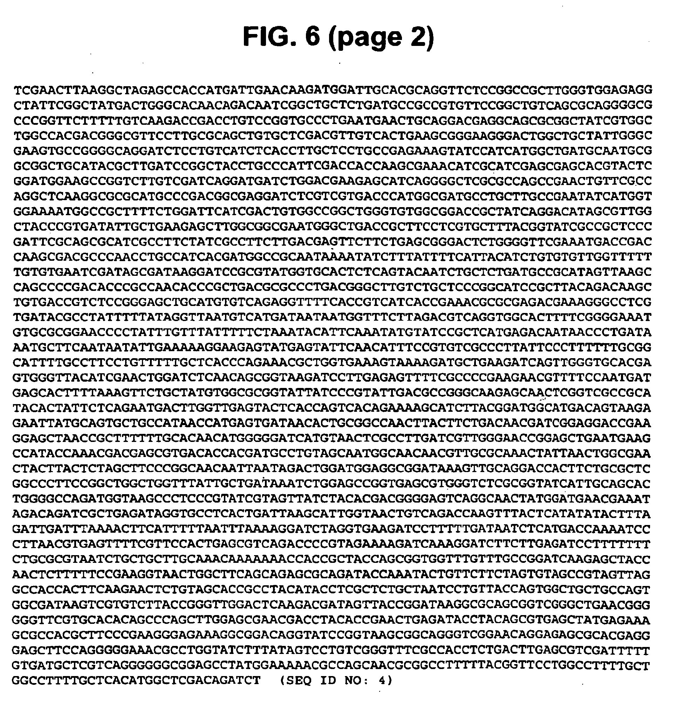

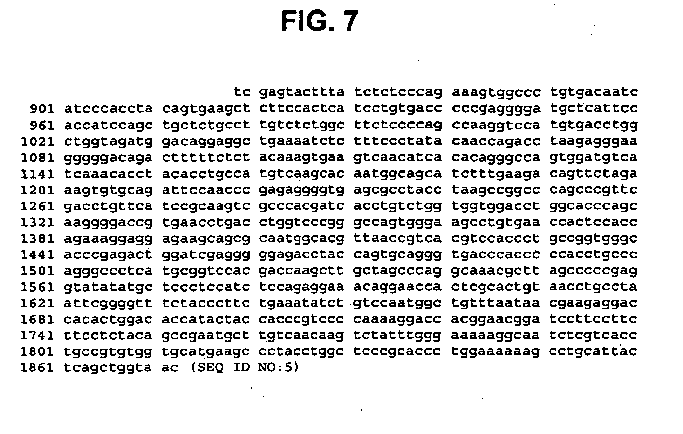

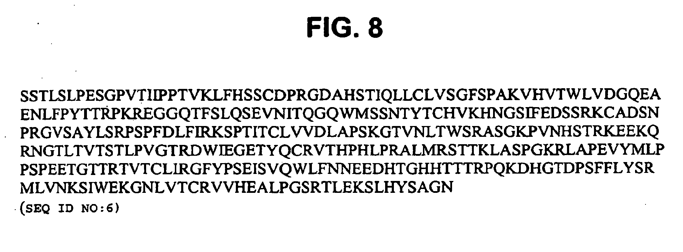

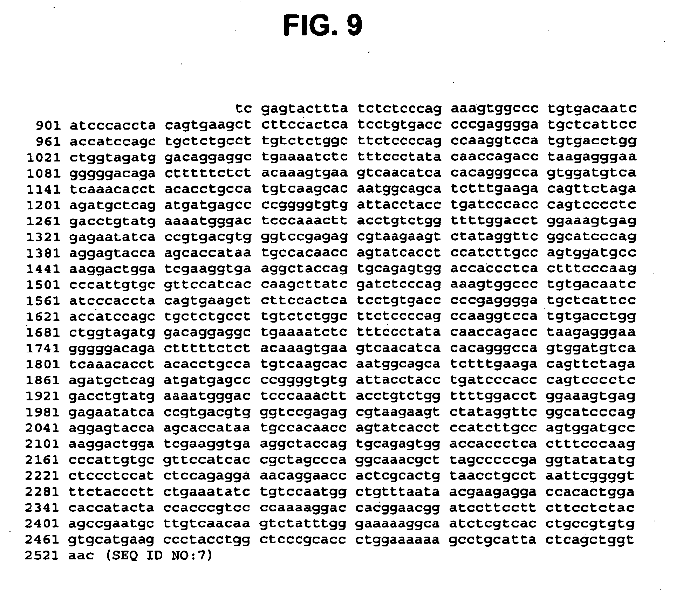

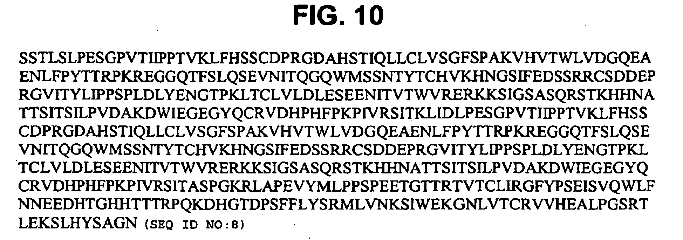

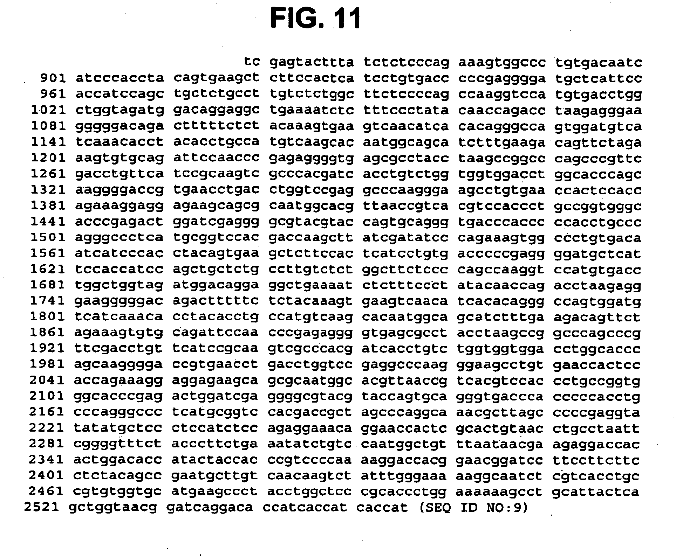

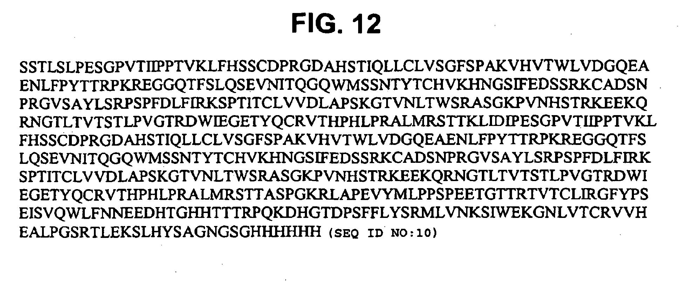

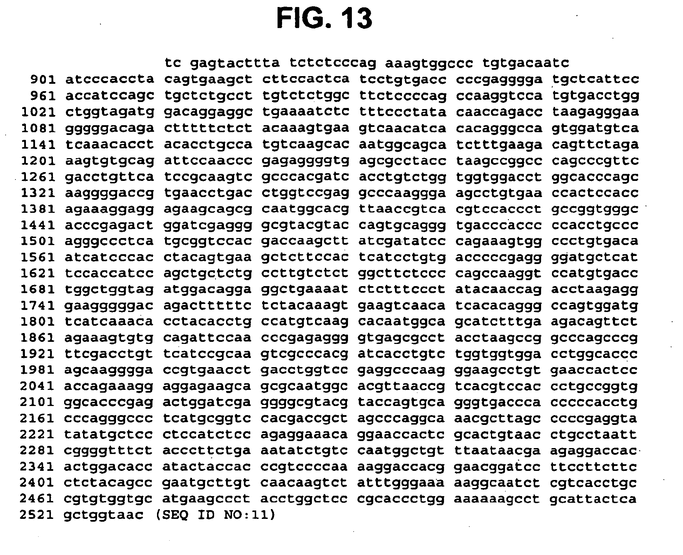

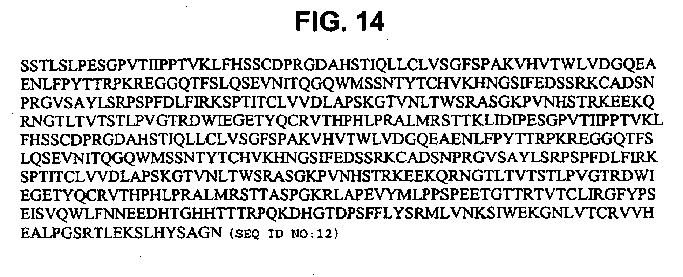

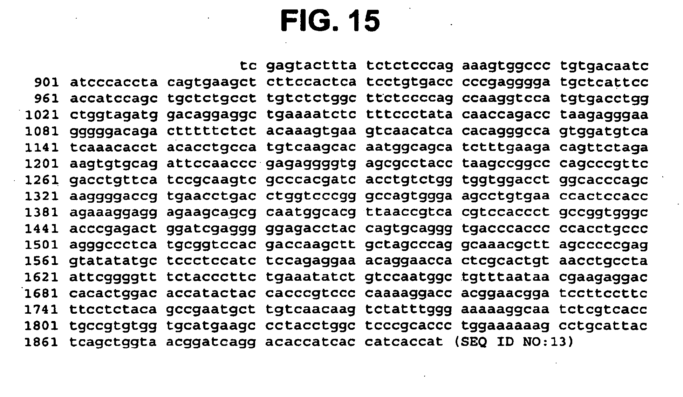

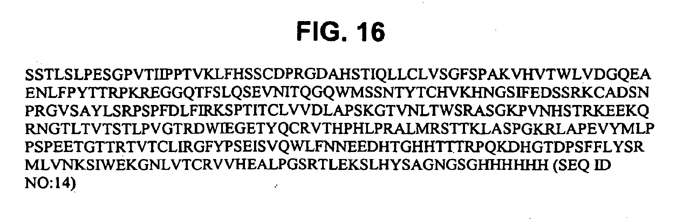

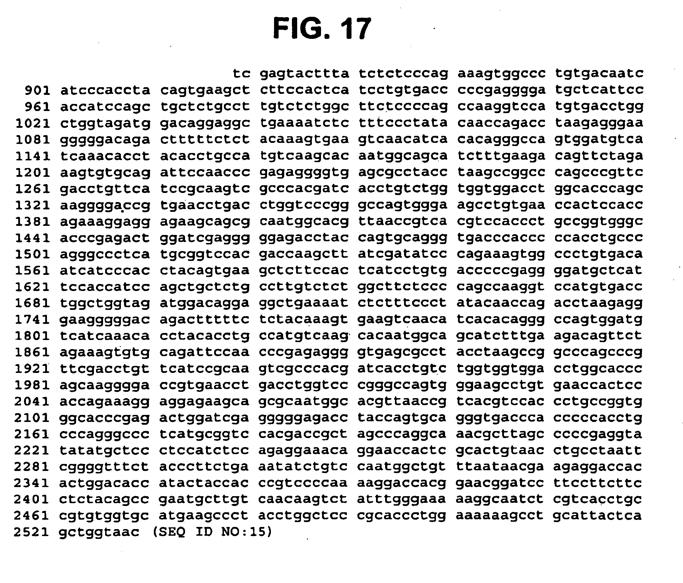

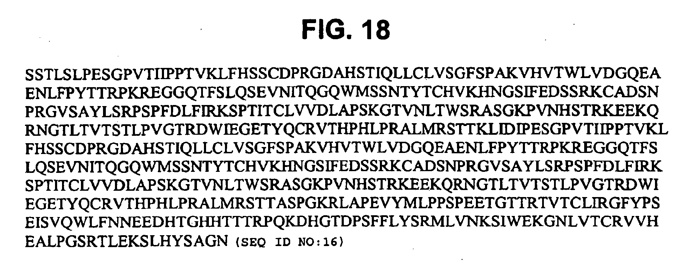

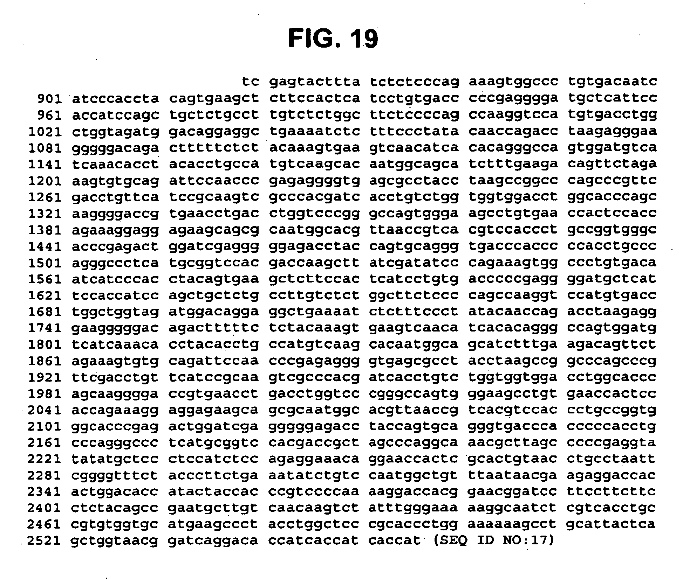

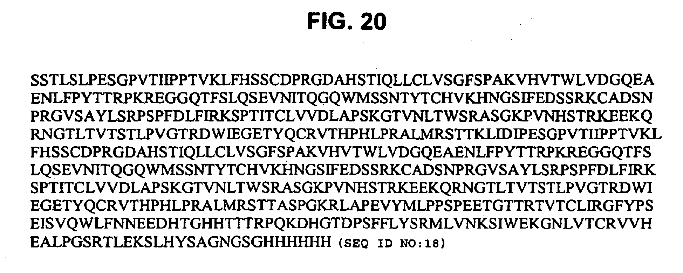

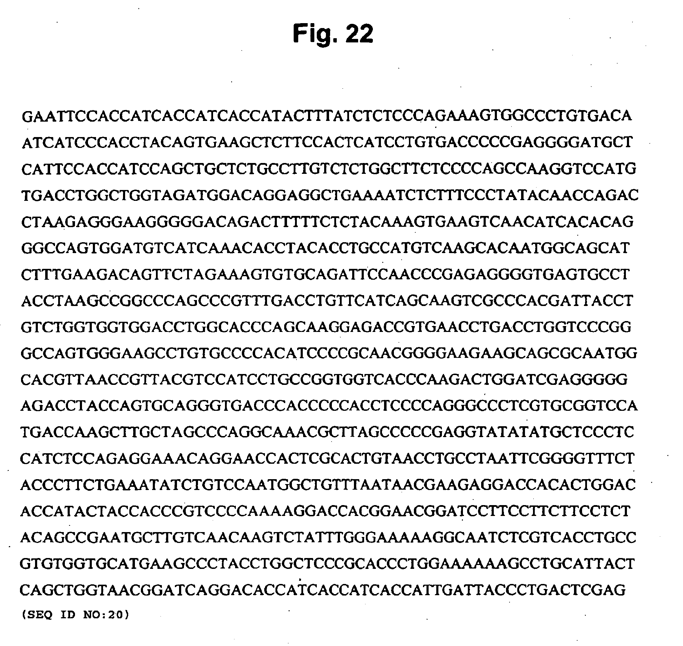

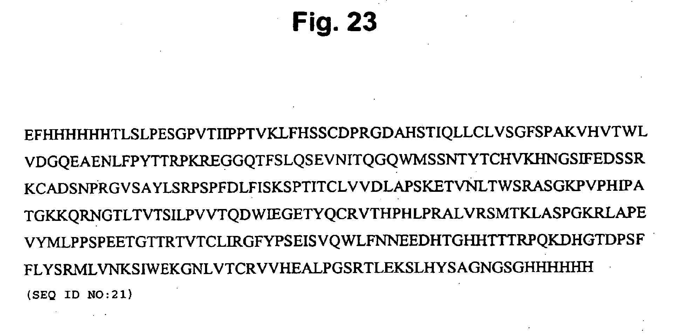

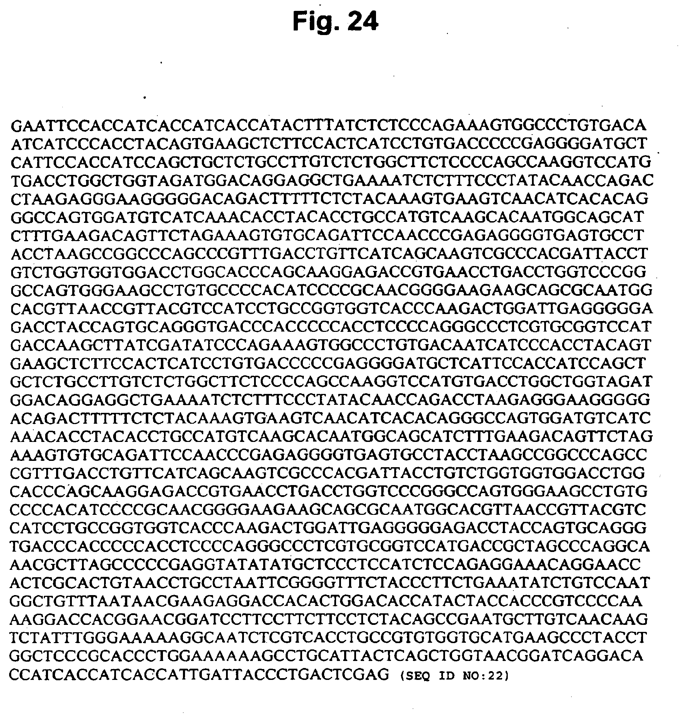

In particular, nucleic acid vectors can be designed to express chimeric IgE polypeptides. Examples of such nucleic acid vectors include, without limitation, those set forth in Figures 1, 2, 5, and 6. In addition, nucleic acid vectors can contain an insert sequence. The term "insert sequence" as used herein refers to a nucleic acid sequence that is inserted into a nucleic acid vector such that that inserted nucleic acid sequence can be expressed. An insert sequence can be a nucleic acid sequence that encodes a chimeric IgE polypeptide such as a polypeptide having the amino acid sequence set forth in Figure 4, 8, 10, 12, 14, 16, 18, 20, or 23. Such nucleic acid sequences can be as set forth in Figure 3, 7, 9, 11, 13, 15, 17, 19, 22, or 24. The term "chimeric IgE polypeptide" as used herein refers to a polypeptide having a combination of IgE sequences (e.g., full domains, half domains, or quarter domains) from different species. A chimeric IgE polypeptide typically contains IgE constant heavy (CH) chain domains (e.g., CH1, CH2, CH3, or CH4). For example, an insert sequence having the sequence set forth in SEQ ID NO:2 can encode an opossum CH2-rat CH3-opossum CH4 (ORO) chimeric IgE polypeptide (SEQ ID NO:3). Other examples of insert sequences include, without limitation, (1) an insert sequence having the sequence set forth in SEQ ID NO:5 that encodes an opossum CH2-human CH3-opossum CH4 (OSO) chimeric IgE polypeptide (SEQ ID NO:6), (2) an insert sequence having the sequence set forth in SEQ ID NO:7 that encodes an opossum CH2-rat CH3-opossum CH2-rat CH3-opossum CH4 (ORORO) chimeric IgE polypeptide (SEQ ID NO:8), and (3) an insert sequence having the sequence set forth in SEQ ID NO:15 that encodes an opossum CH2-human CH3-opossum CH2-human CH3-opossum CH4 (OSOSO) chimeric IgE polypeptide (SEQ ID NO:16). In addition, an insert sequence can have a sequence that encodes any of the polypeptides disclosed in International Patent Application Serial No. PCT/SE99/01896. In addition to rat and human, IgE sequences (e.g., domains) from other species can be used in chimeric insert sequences. Such species include, without limitation, dog, cat, horse, pig, cow, and monkey. For example, an insert sequence including IgE domains from opossum and monkey (e.g., cynomolgus) can encode an opossum CH2-cynomolgus CH3-opossum CH4 (OCO) chimeric IgE polypeptide. Other insert sequences having IgE sequences (e.g., domains) from opossum and monkey include, without limitation, sequences that encode opossum CH2-cynomolgus CH3-opossum CH4 (OCO-H), where the sequence contains a C-terminal histidine-tag; sequences that encode opossum CH2-cynomolgus CH3-opossum CH2-cynomolgus CH3-opossum CH4 (OCOCO); and sequences that encode opossum CH2-cynomolgus CH3-opossum CH2-cynomolgus CH3-opossum CH4, where the sequence contains a C-terminal histidine-tag (OCOCO-H).

An insert sequence can be modified. Such modifications can include, without limitation, additions, deletions, substitutions, point mutations, and combinations thereof. An insert sequence can be modified to include a C-terminal polyhistidine sequence to aid in the purification of the polypeptide encoded by the insert sequence. Polyhistidine sequences used for this purpose have been described elsewhere (Ford et al., Protein Expr. Purif., 2(2-3):95-107, 1991). For example, an insert sequence having the sequence set forth in SEQ ID NO:13 can encode an OSO chimeric IgE polypeptide including a C-terminal polyhistidine sequence (OSO-H; SEQ ID NO:14). An insert sequence can be modified to contain point mutations. For example, an insert sequence having the sequence set forth in SEQ ID NO:11 can encode an OSOSO chimeric IgE polypeptide containing point mutations in the human CH3 domains that abolish mast cell receptor binding (modOSOSO; SEQ ID NO:12). Other examples of modified insert sequences include, without limitation, an insert sequence having the sequence set forth in SEQ ID NO:17 that encodes an OSOSO chimeric IgE polypeptide including a C-terminal polyhistidine sequence (OSOSO-H; SEQ ID NO:18) and an insert sequence having the sequence set forth in SEQ ID NO:9 that encodes an OSOSO chimeric IgE polypeptide including a C-terminal polyhistidine sequence and containing point mutations in the human CH3 domains that abolish mast cell receptor binding (modOSOSO-H; SEQ ID NO:10).

A nucleic acid vector also can contain components that affect the expression of the insert sequence. Examples of such components include, without limitation, promoter, enhancer, leader, and polyadenylation sequences. Such components can be operably linked to the insert sequence. The term "operably linked" as used herein refers to an arrangement where components so described are configured so as to perform their usual function. For example, a nucleic acid vector with an insert sequence encoding an OSOSO chimeric IgE polypeptide also can contain a cytomegalovirus (CMV) promoter sequence (see, for example, Thomson et al., Proc. Natl. Acad. Sci. U. S. A., 81(3):659-663, 1984), an immunoglobulin (Ig) leader sequence (see, for example, Neuberger et al., EMBO J., 2(8):1373-1378, 1983), and a bovine growth hormone (bGH) polyadenylation sequence (see, for example, Goodwin et al., J. Biol. Chem., 267:16330-16334, 1992). In this case, the components can be operably linked to the insert sequence such that the CMV promoter can drive the expression of the insert sequence including the Ig leader sequence and bGH polyadenylation sequence, the Ig leader sequence can direct the expressed insert sequence into the lumen of the endoplasmic reticulum in preparation for secretion, and the bGH polyadenylation sequence can stabilize the insert sequence transcript.

In addition, a nucleic acid vector can contain components that aid in the growth, maintenance, or selection of a host cell containing the nucleic acid vector. Such components include, without limitation, origins of replication and antibiotic selection markers. For example, a nucleic acid vector with a CMV promoter sequence, an Ig leader sequence, an SV40 late polyadenylation sequence, and an insert sequence encoding an OSOSO chimeric IgE polypeptide can also contain an f1 origin of replication sequence, a sequence that confers ampicillin resistance on a bacterial host cell when expressed, and a sequence that confers neomycin resistance on a mammalian host cell when expressed. Other examples of antibiotic selection markers include, without limitation, sequences that confer resistance to hygromycin B, puromycin, kanamycin, tetracycline, blasticidin S, Geneticin^{®}, and zeocin on a host cell when expressed. Nucleic acid vectors that contain one or more than one component described herein can be obtained commercially from, for example, Invitrogen (Carlsbad, CA) and Promega (Madison, WI).

Polypeptide containing self IgE sequences can be obtained using host cells containing a nucleic acid vector (e.g., the pCI-neo vector from Promega, catalogue number E1841) with at least one of the insert sequences provided herein (e.g., ORO, OSO, ORORO, modORORO-H, modOSOSO, OSO-H, OSOSO, and OSOSO-H). Such cells can be prokaryotic cells (e.g., JM109 or DH5α cells) or eukaryotic cells (e.g., NS0, HeLa, BHK-21, COS-7, Sf9, or CHO cells). Host cells containing the nucleic acid vector may or may not express the encoded polypeptide. For example, a host cell may function simply to propagate the nucleic acid vector for use in other host cells. In addition, the nucleic acid vector can be integrated into the genome of the host or maintained in an episomal state. Thus, a host cell can be stably or transiently transfected with the nucleic acid vector.

A host cell can contain a nucleic acid vector with an insert sequence that encodes a chimeric IgE polypeptide. For example, a host cell can contain a nucleic acid vector with an insert sequence encoding an OSO chimeric IgE polypeptide or any of the chimeric IgE polypeptides provided herein. In addition, a host cell can express the polypeptide encoded by the insert sequence.

Various methods can be used to introduce a nucleic acid vector into a host cell *in vivo* or *in vitro.* For example, calcium phosphate precipitation, electroporation, heat shock, lipofection, microinjection, and viral-mediated nucleic acid transfer are common methods that can be used to introduce a nucleic acid vector into a host cell. In addition, naked DNA can be delivered directly to cells *in vivo* as described elsewhere (U.S. Patent Numbers 5,580,859 and 5,589,466). Further, a nucleic acid vector can be introduced into cells to generate transgenic animals.

Transgenic animals can be aquatic animals (such as fish, sharks, dolphin, and the like), farm animals (such as pigs, goats, sheep, cows, horses, rabbits, and the like), rodents (such as rats, guinea pigs, and mice), non-human primates (such as baboon, monkeys, and chimpanzees), and domestic animals (such as dogs and cats). Several techniques known in the art can be used to introduce a nucleic acid vector into animals to produce the founder lines of transgenic animals. Such techniques include, without limitation; pronuclear microinjection (U.S. Patent No. 4,873,191); retrovirus mediated gene transfer into germ lines (Van der Putten et al., Proc. Natl. Acad. Sci., USA, 82:6148 (1985)); gene transfection into embryonic stem cells (Gossler A et al., Proc Natl Acad Sci USA 83:9065-9069 (1986)); gene targeting into embryonic stem cells (Thompson et al., Cell, 56:313 (1989)); nuclear transfer of somatic nuclei (Schnieke AE et al., Science 278:2130-2133 (1997)); and electroporation of embryos (Lo CW, Mol. Cell. Biol., 3:1803-1814 (1983)). Once obtained, transgenic animals can be replicated using traditional breeding or animal cloning.

Various methods can be used to identify a host cell containing a nucleic acid vector provided herein. Such methods include, without limitation, PCR, nucleic acid hybridization techniques such as Northern and Southern analysis, and *in situ* nucleic acid hybridization. In some cases, immunohistochemistry and biochemical techniques can be used to determine if a cell contains a nucleic acid vector with a particular insert sequence by detecting the expression of a polypeptide encoded by that particular insert sequence.

Any method can be used to produce recombinant chimeric IgE polypeptides. Such methods involve culturing a host cell that expresses a chimeric IgE polypeptide and recovering the expressed chimeric IgE polypeptides. Any method can be used to recover a recombinant chimeric IgE polypeptide. For example, recombinant chimeric IgE polypeptides that are present in a host cell homogenate can be recovered using ion exchange chromatography. In another example, recombinant chimeric IgE polypeptides with polyhistidine sequences can be recovered from a host cell homogenate by passing the homogenate over a nickel column and eluting the polyhistidine-containing polypeptides with imidazole. A particular recombinant chimeric IgE polypeptide with a leader sequence that directs that polypeptide's secretion can be recovered from the growth medium of a host cell expressing that polypeptide. For example, the growth medium from a culture of mammalian host cells expressing and secreting ORO or OSO polypeptides can be collected, and the ORO or OSO polypeptides can be recovered using chromatography. It is understood that a leader sequence that directs the secretion of a polypeptide typically is removed from that polypeptide in the host cell by proteolysis. Thus, the recovered secreted polypeptide, in many cases, is free of any translated leader sequence.

In one embodiment, the cell medium from a clonal CHO cell line expressing and secreting ORO or OSO polypeptides is collected and centrifuged to remove cell debris. After centrifuging, the supernatant is dialyzed and passed over an ion exchange column allowing the ORO or OSO polypeptides to bind. The bound ORO or OSO polypeptides are eluted using a sodium chloride/sodium acetate gradient, and the eluted fractions are screened for recombinant ORO or OSO polypeptides using an ELISA technique. The eluted fractions with high ELISA reactivity can be pooled and dialyzed again, and the dialyzed pooled fractions can be passed over a hydrophobic interaction column allowing the ORO or OSO polypeptides to bind. The bound ORO or OSO polypeptides are eluted using a sodium phosphate gradient, and the eluted fractions are again screened for recombinant ORO or OSO polypeptides using an ELISA technique. The eluted fractions with high ELISA reactivity can be further analyzed by silver stained SDS-PAGE to estimate the purity of the ORO or OSO polypeptides.

As described herein, alum as well as other aluminum-based compounds (e.g., Al₂O₃) can be combined with a polypeptide containing a self polypeptide segment (e.g., a self IgE sequence) to form a composition that elicits an anti-self response when administered to a mammal. Aluminum-based compounds can be obtained from various commercial suppliers. For example, REHYDRAGEL^{®} adjuvants can be obtained from Reheis Inc. (Berkeley Heights, NJ). REHYDRAGEL^{®} adjuvants are based on crystalline aluminum oxyhydroxide, and are hydrated gels containing crystalline particles with a large surface area (about 525 m²/g). Their Al₂O₃ content typically ranges from about 2 percent to about 10 percent. Rehydragel LG, for example, has an Al₂O₃ content of about 6 percent, and flows readily upon slight agitation. Rehydragel LG also has a protein binding capacity of 1.58 (i.e., 1.58 mg of bovine serum albumin bound per 1 mg of Al₂O₃), a sodium content of 0.02 percent, a chloride content of 0.28 percent, undetectable sulphate, an arsenic level less than 3 ppm, a heavy metal content less than 15 ppm, a pH of 6.5, and a viscosity of 1090 cp. Rehydragel LG can be combined with a polypeptide solution (e.g., a polypeptide in PBS) to yield Al(OH)₃₋ In addition, ALHYDROGEL^{™}, an aluminum hydroxy gel adjuvant, (Alhydrogel 1.3%, Alhydrogel 2.0%, or Alhydrogel "85") obtained from Brenntag Stinnes Logistics can be used.

In addition, MN51 can be combined with a polypeptide containing a self polypeptide segment (e.g., a self IgE sequence) to form a composition that elicits an anti-self response when administered to a mammal. MN51 (MONTANIDE^{®} Incomplete SEPPIC Adjuvant (ISA) 51) as well as MN720 are available from Seppic (Paris, France). MN51 contains mannide oleate (MONTANIDE^{®} 80, also known as anhydro mannitol octadecenoate) in mineral oil solution (Drakeol 6 VR). MONTANIDE^{®} 80 is a limpid liquid with a maximum acid value of 1, a saponification value of 164-172, a hydroxyl value of 89-100, an iodine value of 67-75, a maximum peroxide value of 2, a heavy metal value less than 20 ppm, a maximum water content of 0.35%, a maximum color value of 9, and a viscosity at 25°C of about 300 mPas. MONTANIDE^{®} associated with oil (e.g., mineral oil, vegetable oil, squalane, squalene, or esters) is known as MONTANIDE^{®} ISA. Drakeol 6 VR is a pharmaceutical grade mineral oil. Drakeol 6 VR contains no unsaturated or aromatic hydrocarbons, and has an A.P.I. gravity of 36.2-36.8, a specific gravity at 25°C of 0.834-0.838, a viscosity at 100°F of 59-61 SSU or 10.0-10.6 centistokes, a refractive index at 25°C of 1.458-1.463, a better than minimum acid test, is negative for fluorescence at 360 nm, is negative for visible suspended matter, has an ASTM pour test value of 0-15°F, has a minimum ASTM flash point of 295°F, and complies with all RN requirements for light mineral oil and ultraviolet absorption. MN51 contains about 8 to 12 percent anhydro mannitol octadecenoate and about 88 to 92 percent mineral oil. MN51 is a clear yellow liquid having a maximum acid value of 0.5, a saponification value of 16-20, a hydroxyl value of 9-13, a maximum peroxide value of 2, an iodine value of 5-9, a maximum water content of 0.5 percent, a refractive index at 25°C between 1.455 and 1.465, a density at 20°C of about 0.85, and a viscosity at 20°C of about 50 mPaS. The conductivity of a 50:50 mixture of MN51 and saline is less than 10 µScm⁻¹.

Other adjuvants include immuno-stimulating complexes (ISCOMs) that can contain such components as cholesterol and saponins. ISCOM matrices can be prepared and conjugated to Cu²⁺ using methods such as those described herein. Adjuvants such as FCA, FIA, MN51, MN720, and Al(OH)₃ are commercially available from companies such as Seppic, Difco Laboratories (Detroit, MI), and Superfos Biosector A/S (Vedbeak, Demark).

In some embodiments, a composition also can contain one or more additional immunostimulatory components. These include, without limitation, muramyldipeptide (e.g., N-acetylmuramyl-L-alanyl-D-isoglutamine; MDP), monophosphoryl-lipid A (MPL), and formyl-methionine containing tripeptides such as N-formyl-Met-Leu-Phe. Such compounds are commercially available from Sigma Chemical Co. (St. Louis, MO) and RIBI ImmunoChem Research, Inc. (Hamilton, MT), for example.

A "unit dose" of a composition refers to the amount of a composition administered to a mammal at one time. A unit dose of the compositions provided herein can contain any amount of polypeptide. For example, a unit dose of a composition can contain between about 10 µg and about 1 g (e.g., 10 µg, 15 µg, 25 µg, 30 µg, 50 µg, 100 µg, 250 µg, 280 µg, 300 µg, 500 µg, 750 µg, 1 mg, 10 mg, 15 mg, 25 mg, 30 mg, 50 mg, 100 mg, 250 mg, 280 mg, 300 mg, 500 mg, 750 mg, or more) of a polypeptide. In some embodiments, the polypeptide can be dissolved or suspended in a physiological buffer such as, for example, water or phosphate buffered saline (PBS), pH 7.0. The solution of polypeptide then can be combined with the adjuvant and any other components of the composition.

Similarly, a unit dose of a composition can contain any amount of an adjuvant. For example, a unit dose can contain between about 10 µL and about 1 mL (e.g., 10 µL, 25 µL, 50 µL, 100 µL, 250 µL, 500 µL, 750 µL, 800 µL, 900 µL, or 1 mL) of one or more adjuvants. In addition, a unit dose of a composition can contain any amount of another immunostimulatory component. For example, a composition provided herein can contain between about 10 µg and about 1 g (e.g., 10 µg, 15 µg, 25 µg, 30 µg, 50 µg, 100 µg, 250 µg, 280 µg, 300 µg, 500 µg, 750 µg, 1 mg, 10 mg, 15 mg, 25 mg, 30 mg, 50 mg, 100 mg, 250 mg, 280 mg, 300 mg, 500 mg, 750 mg, or more) of an immunostimulatory component.

The compositions provided herein can contain any ratio of adjuvant to polypeptide. The adjuvant:antigen ratio can be 50:50 (vol:vol), for example. Alternatively, the adjuvant:antigen ratio can be, without limitation, 90:10, 80:20, 70:30, 64:36, 60:40, 55:45, 40:60, 30:70, 20:80, or 90:10.

The invention also provides methods for preparing the compositions provided herein. Such methods can involve suspending an amount of a polypeptide (e.g., 100 µg of ORO) in a suitable amount of a physiological buffer (e.g., 50 µL of PBS pH 7.0), and then combining the suspended or dissolved antigen with a suitable amount of an adjuvant (e.g., 50 µL of MN51 or 100 µL of REHYDRAGEL^{®}). The combining step can be achieved by any method, including stirring, shaking, vortexing, or passing back and forth through a needle attached to a syringe, for example. It is noted that the composition can be prepared in batch, such that enough unit doses are obtained for multiple injections (e.g., injections into multiple animals or multiple injections into the same animal).

The invention also provides methods for inducing an anti-self response in a mammal (e.g., a mouse, a rat, a cat, a dog, a horse, a cow, a non-human primate such as a cynomolgus monkey, or a human). Such methods can involve administering to a mammal a composition provided herein, wherein the composition contains a polypeptide that includes an amino acid sequence from a self polypeptide (e.g., an amino acid sequence from the CH3 domain of an IgE polypeptide found in that particular species of mammal). The polypeptide can contain at least one amino acid sequence from another species (e.g., an amino acid sequence from the CH2 or CH4 domain of an IgE polypeptide found in a different species).

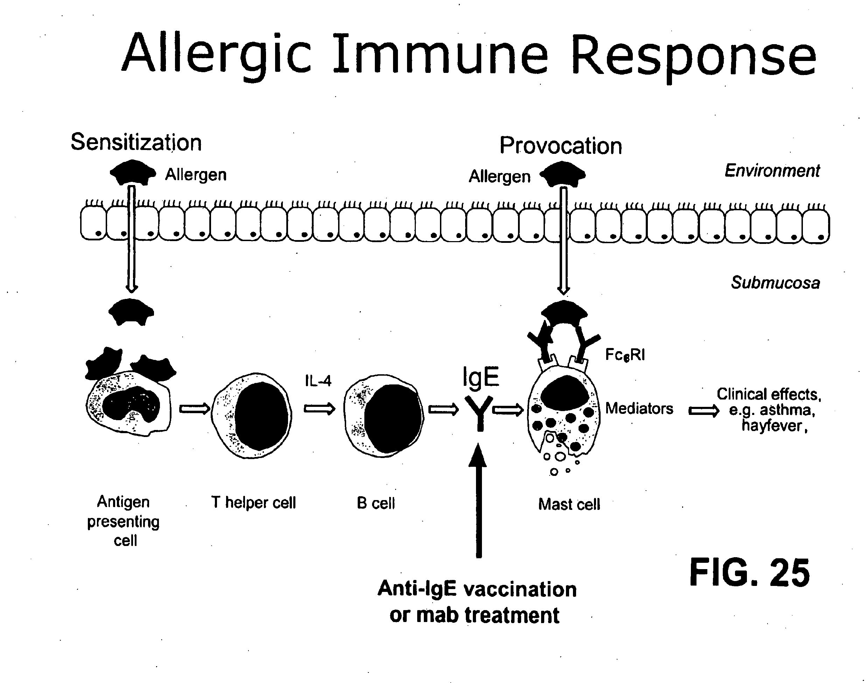

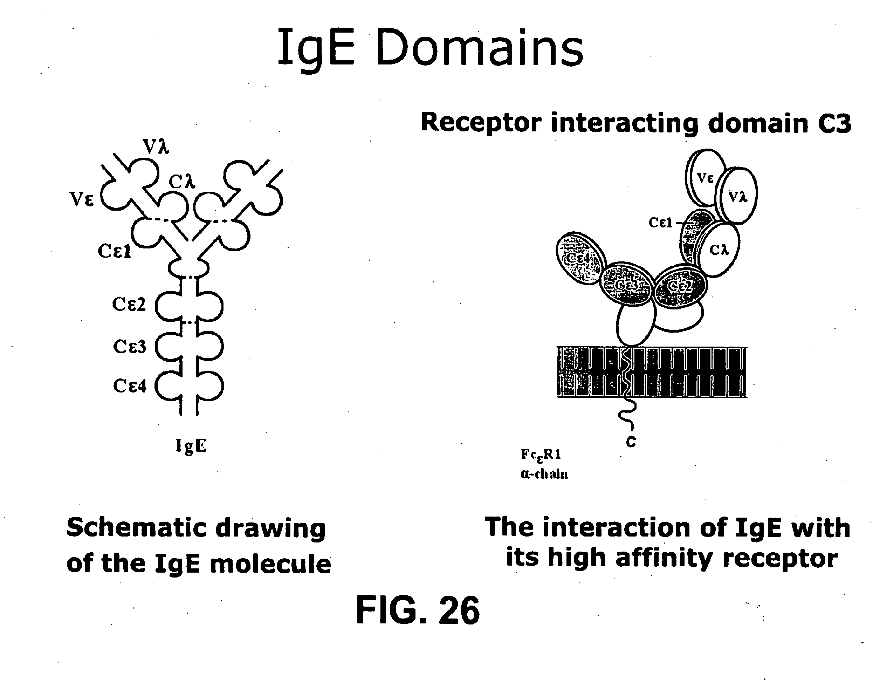

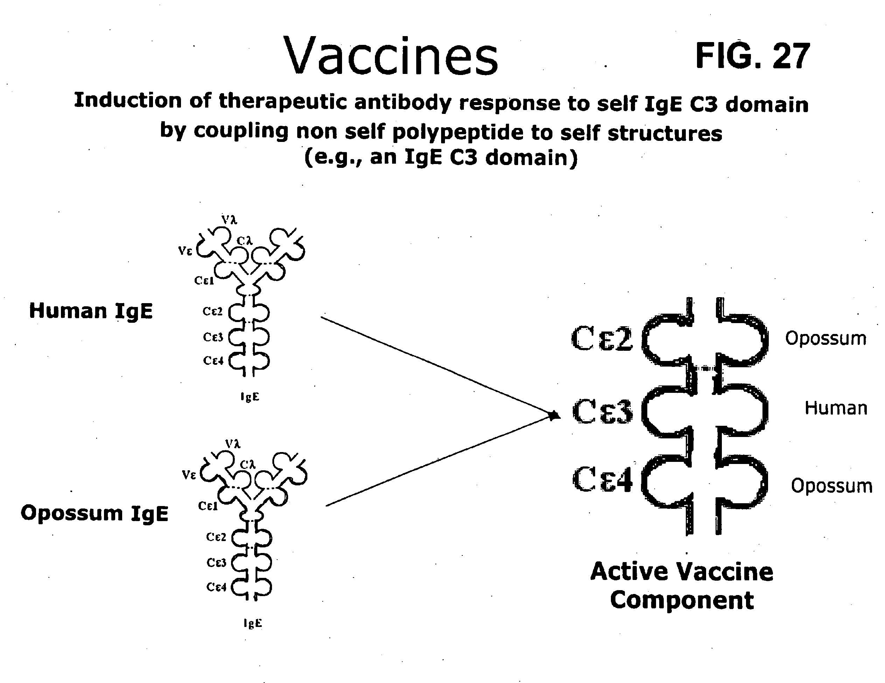

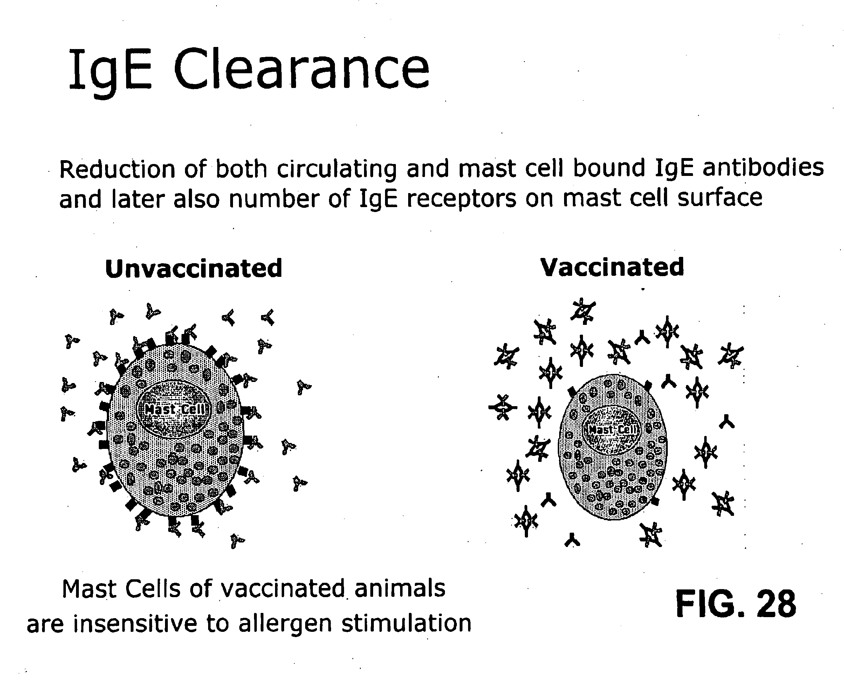

In general, compositions containing a polypeptide provided herein can be used as an allergy vaccine to abrogate the allergic cascade by eliminating circulating IgE (Figures 25-28). The compositions can induce an antibody response against self-IgE in the recipient. Although not limited to any particular mode of action, it is believed that administration of compositions containing a polypeptide with self IgE sequences in a context which allows the mammal's tolerance to IgE to be broken leads to the production of anti-self IgE antibodies, which in turn decreases the level of circulating self IgE antibodies.

The compositions provided herein can be administered by a number of methods. Administration can be, for example, topical (e.g., transdermal, ophthalmic, or intranasal); pulmonary (e.g., by inhalation or insufflation of powders or aerosols); oral; or parenteral (e.g., by subcutaneous, intrathecal, intraventricular, intramuscular, or intraperitoneal injection, or by intravenous drip). Administration can be rapid (e.g., by injection) or can occur over a period of time (e.g., by slow infusion or administration of slow release formulations).

Any dose can be administered to a mammal. Dosages can vary depending on the relative potency of individual compositions, and can generally be estimated based on data obtained from *in vitro* and *in vivo* animal models. Typically, dosage is from about 0.01 µg to about 100 g per kg of body weight, and may be given once or more daily, weekly, or even less often. Following successful administration, it may be desirable to have the subject undergo additional booster administrations to maintain a suitable level of the anti-self response.

The anti-self response (e.g., anti-self IgE antibody response) to a composition in a mammal can be assessed using any method. For example, the anti-self IgE titer can be measured. Alternatively, a "titer dilution₅₀ value" can be determined by using an ELISA and measuring the optical density (OD) of dilutions (e.g., serial dilutions) of the serum samples. The dilution factor that results in a 50 percent reduction from the maximal OD is considered to be the titer dilution₅₀ value. This value can be calculated by curve fitting using; for example, the SOFTmax^{®} Pro 4.0 software program that is available from Molecular Devices, Inc. (Sunnyvale, CA). Using a four parameter non-linear regression for curve fitting, this program can be used to fit data points to a curve and determine the titer dilution₅₀ value.

The invention also provides methods for measuring free IgE levels in the serum of a subject (e.g., a mammal) treated with a polypeptide containing one or more self IgE segments (e.g., ORO). Such methods can involve providing a serum sample from a subject treated with, for example, ORO, and incubating the sample with an IgE receptor polypeptide such as the human IgE receptor alpha-chain (e.g., the polypeptide having GenBank^{®} Accession No. NM_002001) to form IgE/IgE receptor complexes. Any IgE receptor sequence (or portion thereof) can be used. For example, a human IgE receptor alpha-chain can be used to measure free IgE in humans or other primates such as monkeys. After incubating the IgE receptor polypeptide with the sample containing free IgE, the formed IgE/IgE receptor complexes can be measured. Any method can be used to measure IgE/IgE receptor complexes. For example, immunological assays such as ELISAs and ELISA-like procedures can be used to measure IgE/IgE receptor complexes.

The invention also provides kits for assessing the amount of free IgE present in a mammal treated with an anti-self IgE polypeptide-containing composition. Such kits can contain an IgE receptor sequence and an antibody capable of binding to an IgE/IgE receptor complex. The kits provided herein also can contain a composition described herein such as an ORO-containing composition. Such kits can be used to assess free IgE levels in a mammal and, if needed, to provide an additional booster of the self polypeptide-containing composition. The kits provided herein can contain additional reagents such as IgE standards, negative controls, enzyme preparations, and enzyme substrates.

The invention will be further described in the following examples, which do not limit the scope of the invention described in the claims.

### EXAMPLES

### Example 1 - Evaluation of vaccine adjuvants

### Production of the active vaccine component, H-ORO

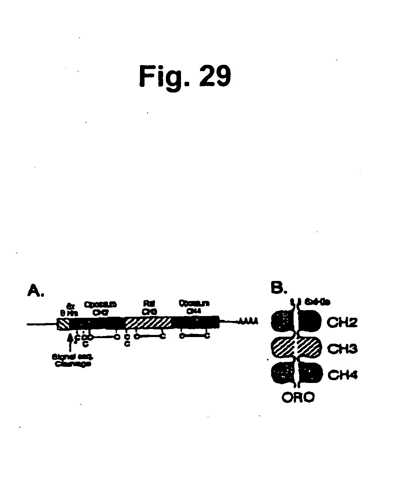

The active component in the vaccine, H-ORO, was encoded by a recombinant construct containing 1041 bp from the C3 domain of rat ε-heavy chain (Hellman et al., Nucl. Acids Res., 10:6041 (1982)) flanked by the C2 and C4 domains of the opossum ε-heavy chain (Aveskogh and Hellman, Eur. J. Immunol., 28:2738 (1998)). This construct (Figure 29) was expressed in 293-EBNA cells and purified on Ni-NTA Agarose (QIAGEN GmbH, Germany) as described previously (Vemersson et al., FASEB J., 16:875 (2002)). The H-ORO component was obtained at a concentration of 1.5 mg/mL in PBS pH 7.0.

### Study 1:

Twenty, 8-10 week old female Wistar rats (Benton and Kingman, Sollentuna, Sweden) were sensitized against ovalbumin (OVA). The animals received an initial intraperitoneal (i.p.) injection of 10 µg OVA (Sigma Chemical Co., MO) in PBS pH 7.0, followed by weekly i.p. injections of 3 µg OVA in PBS pH 7.0 for 5 weeks prior to the initial vaccination, and continuing throughout the vaccination program.

Five groups of four animals received an initial i.p. vaccination ofH-ORO mixed with one of the following adjuvants: FCA (Difco Laboratories, Detroit, MI), Al(OH)₃ (Superfos Biosector A/S, Vedbeak, Denmark), or Cu²⁺-conjugated ISCOM matrix (Andersson et al. (2001) J. Immunol. Methods 255:135). Animals 1-4 received 100 µg H-ORO in 50 µL PBS pH 7.0, mixed 50:50 with FCA. Animals 5-8 received 100 µg H-ORO and 10 vol% Al(OH)₃ slurry in 100 µL PBS pH 7.0. Animals 9-12 received 100 µg H-ORO in 50 µL PBS pH 7.0, mixed 50:50 with Cu²⁺-conjugated ISCOM matrix. Animals 13-16 received 25 µg H-ORO in 50 µL PBS pH 7.0, mixed 50:50 with Cu²⁺-conjugated ISCOM matrix. Booster vaccinations were administered in week three of the treatment program. The booster vaccinations were identical to the initial vaccinations, with the exception that FIA (Difco Laboratories) was used instead of FCA in animals 1-4.

Blood samples of 1 mL were collected from the tail vein before initiating sensitization, three days prior to vaccination, and two weeks after the booster vaccination. The blood was allowed to coagulate overnight at 4°C and spun down for 10 minutes at 10,000 rpm (EBA12R, Hettich Zentrifugen, Germany). The sera were transferred to Eppendorf tubes and frozen until evaluation by ELISA.

The vaccine preparations containing Al(OH)₃ were mixed to a 10 vol% Al(OH)₃ slurry with 100 µg H-ORO protein in PBS pH 7.0 one day prior to vaccination, and stored at 4°C overnight. The ISCOM matrix was prepared as follows: IDA Matrix with Cu²⁺ had an estimated QA content of 1.7 mg/mL and an estimated cholesterol content of 0.5 mg/mL (Prep. 990823 B). Matrix without Cu²⁺ had a QA content of 2.6 mg/mL, and cholesterol was estimated to be 0.8 mg/mL (Prep. 990320). To load the matrix with Cu²⁺; a stock solution of 1 M CuSO₄*5H₂O in water was prepared. This solution was added to the matrix preparation to a final concentration of 0.1 M Cu²⁺. The mixture was incubated on a shaker in room temperature for 30 minutes, followed by dialysis against PBS overnight at 4°C. Protein antigen was added in a ratio of 1:1 to the cholesterol content and incubated at 4°C overnight.

### Study 2:

A total of forty, 8-10 weeks old female Wistar rats divided into ten groups of four animals received an initial 200 µL i.p. injection of 100 µg H-ORO in PBS pH 7.0, together with an adjuvant and in some groups, additional immunostimulators. Animals 1-4 received FCA mixed at a 50:50 ratio with antigen. Animals 5-8 received MN51 (Seppic, Paris Cedex 07, France) at a 50:50 ratio to the antigen. Animals 9-12 were injected with MN51:antigen (50:50) and 200 µg MDP (Sigma Chemical Co.) per animal (25 µg of MDP was dissolved in sterile PBS to a final concentration of 10 mg/mL). Group 4, animals 13-16, received MN51 :antigen (50:50) and 200 µg MPL. A 10 mg/mL solution of MPL (RIBI ImmunoChem Research, Inc., Hamilton, MT) in methanol/chloroform (1:4) was aliquoted in volumes corresponding to doses of 0.2 mg and 0.1 mg MPL per animal and evaporated. Animals 17-20 were given MN51:antigen (50:50), 200 µg MDP, and 100 pg MPL. Animals 21-24 received MN51:antigen (50:50), 200 µg MDP, 100 µg MPL, and 100 µg fMLP (Sigma Chemical Co.). Ten mg of fMLP was dissolved in 1 mL sterile PBS and 1 mL 95% ethanol. Animals 25-28 received MN720 (Seppic) in a 70:30 ratio with H-ORO. Animals 29-32 were injected with MN720:antigen (64:36) and 200 µg MDP per animal. Animals 33-36 were injected with MN720:antigen (70:30) and 200 µg MPL. Group 10, animals 37-40, were given MN720:antigen (64:36) with the addition of 200 µg MDP and 100 µg MPL.

The booster dose contained half the amount of H-ORO (50 µg) given in the initial vaccination, and FIA was used instead of FCA. Blood was drawn from the tail vein ten days prior to vaccination and two weeks after the booster. The blood was treated as described in Study 1.

### Anti rat-IgE ELISA:

The anti rat-IgE ELISA has been described previously *(Vemersson et al., supra*). The samples were assayed in singles and horse sera served as the assay blank. In order to correlate the values, serial dilutions of two of the samples were assayed on every plate.

### Anti Opossum C2-C3-C4 ELISA:

The same procedure as above (Vemersson *et al., supra*) was used, except for the coating antigen, which in this case was opossum C2C3C4 at a concentration of 5 µg/mL in carbonate buffer pH 9.6.

### The H-ORO component:

To address the question of difference in immune response against self and non-self components, a hybrid molecule containing both self and non-self regions was designed and produced as a recombinant protein. A vaccine containing the third constant domain from the rat ε-heavy chain (the target species) flanked by the second and fourth constant domains of the American opossum IgE heavy chain (OpossumCH2-RatCH3-OpossumCH4; H-ORO, Figure 29) was expressed in 293-EBNA human embryonic kidney cells. The average yield was about 1 mg H-ORO protein per liter conditioned media. Based on SDS-PAGE, the purity of H-ORO was estimated to be at least 90%, and the major contaminant was identified as BSA derived from the FBS-supplemented cell culture medium (Vernersson *et al., supra*). The opossum sequences differed in sequence by almost 60% from rat IgE, and thereby served as a non-self component. The opossum domains had two additional functions, acting both as structural support for the self-component (the C3 domain) and to break T cell tolerance to the self component by providing foreign T cell epitopes.

As a reagent for measuring anti opossum responses by ELISA, a recombinant opossum C2C3C4 IgE was produced (OOO) by the same procedure as described above. Purified whole rat IgE was used for measurements of the anti rat-IgE C3 responses.

### Results:

Three adjuvants were studied: Freund's adjuvant, Alum (Al(OH)₃), and a preparation of ISCOMs. The various adjuvants were administered by i.p. injection together with the H-ORO vaccine component. The animals were divided into four groups: four animals were given 100 µg of H-ORO in CFA, four animals received the same amount of protein absorbed to Alum (a 10 vol% Al(OH)₃ slurry) from a commercially available preparation, four animals received 100 µg of H-ORO absorbed to the surface of 100 µg of ISCOMs, and the last four animals were administered the same amount of ISCOMs but only 25 µg of H-ORO. The rationale behind the reduced levels of antigen was to study the effect of a lower loading density on the ISCOMs, which may influence the availability for the immune system to recognize the surface epitopes.

Booster vaccinations were administered in week three of the treatment program. The booster vaccinations were identical to the initial vaccinations, with the exception that IFA was used instead of FCA in animals 1-4. Blood samples of 1 mL were collected from the tail vein three days prior to vaccination and two weeks after the booster vaccination.

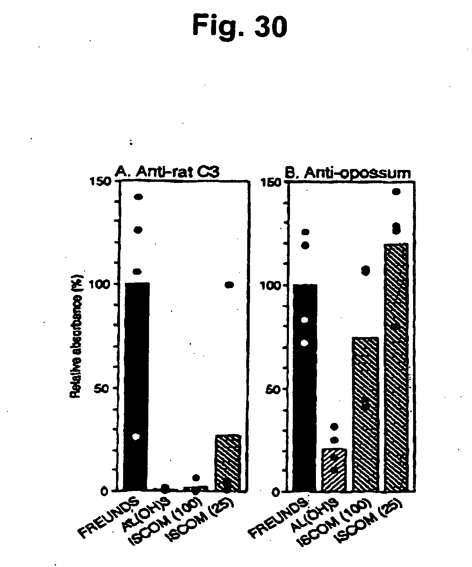

Comparative ELISA analyses were performed on sera from week 5 of the treatment program. The ELISA plates were either coated with whole rat IgE in order to measure anti rat C3 immune responses (the anti-self-response), or with opossum C2C3C4 recombinant protein (OOO) to measure anti non-self responses. Surprisingly, substantial anti-self-responses were detected only with Freund's adjuvant (Figure 30A). No response was detected with Alum in this experiment, and a response was observed only in one of the four animals that received the 25 µg dose of H-ORO absorbed on ISCOMs. However, when measuring the anti non-self responses, the ISCOMs were comparable with Freund's, and no significant difference in magnitude between these two adjuvants could be detected. However, Alum was shown to be a less potent adjuvant. Alum gave a response of approximately 20% of the levels seen with Freund's and the ISCOMs (Figure 30B).

The relative difference between the self and the non-self responses also was estimated by performing an ELISA assay in which different wells on the same plate were coated with either rat IgE or OOO. The ratios between anti non-self response and the anti-self-response in the Freund's treated animals were found to be 150, 175, 150 and 750 for the four animals, giving a mean value of approximately 300 times difference in titer. Although a substantial induction of anti-self-antibodies was observed, this suggests that the titers of antibodies against self IgE sequences were substantially lower than the titers of antibodies against the non-self IgE sequences.

Although the induction of anti non-self immune responses with the ISCOM preparation was comparable in magnitude to the levels obtained in the animals given Freund's adjuvant, the anti-self-IgE titers were undetectable or very low. In addition, no detectable anti-self IgE was detected with the Alum preparation. The only adjuvant that resulted in significant levels of anti-self IgE antibodies was Freund's adjuvant, an adjuvant based on mineral oil.

### Example 2 - A comparative analysis of MN51 and MN720

One commercially available mineral oil adjuvant (MN51) and one adjuvant (MN720) that is based on plant oil but has the same emulsifier (mannide monooleate) as MN51 were tested to compare their effects with the effects of Freund's adjuvant.

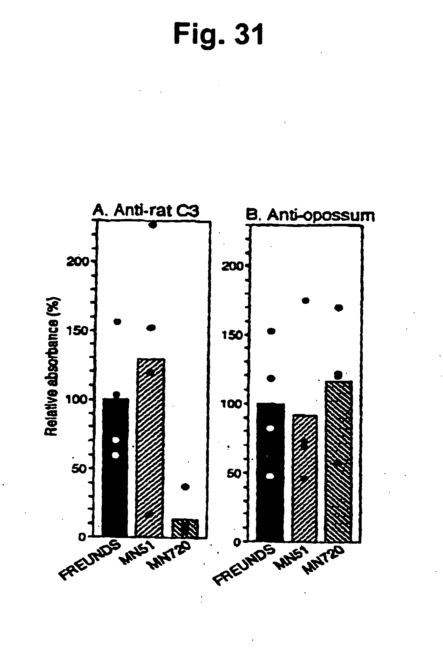

As in the first experiment, four animals in each group were tested for the induction of anti-self and anti non-self responses. The amounts of antigen and adjuvant were 100 µL of adjuvant and 100 µg of H-ORO. Comparative ELISA analyses were performed using sera obtained in week 5 of the treatment program. A substantial anti-self IgE response was detected in all animals. The most prominent response, however, was seen with MN51, which actually was slightly higher (130%) than the response observed with Freund's adjuvant (Figure 31A). MN720 produced a response corresponding to only about 15% of the response seen with Freund's. In contrast to the observation for the anti-self-response, however, all three adjuvants were almost equally potent in their abilities to induce an anti non-self response (Figure 31B).

The relative magnitudes of the anti-non-self and anti-self responses also were determined. The ratio between the anti non-self response and the anti-self-response in the Freund's treated animals was 50, 65, and 75 for three of the four animals, giving a mean value of about 63 times difference in titer. The amount of sera obtained from the fourth animal was insufficient to conduct this analysis. For MN51, the ratios were 200, 30, 40, and 26 for the four animals, giving a mean value of 74 times difference in titer.

### Example 3 - An analysis of potential additive effects with MDP, Lipid A, and formyl-Met polypeptides

Based on the results from the two previous experiments, it was concluded that the mineral oil based adjuvants are the most effective in inducing anti-self-IgE responses. The difference between anti-self and anti non-self responses can still be quite substantial, however, and probably frequently exceeds a 50-fold difference. Bacterial immunostimulatory substances thus were tested in order to determine whether an additive effect could be achieved. A series of experiments were conducted in which rats were immunized in groups of four with 100 µg H-ORO in MN51 or MN720, with addition of either 200 µg of MDP, 200 µg of MPL, 200 µg of MDP and 100 µg of MPL, or 200 µg of MOP, 100 µg of MPL, and 100 µg of fMLP.

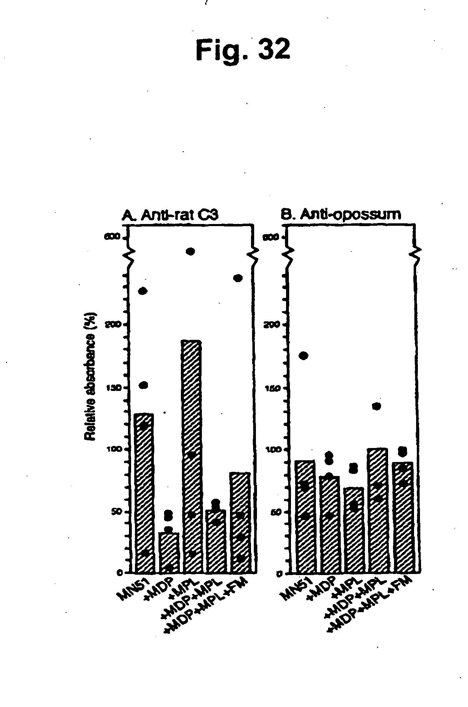

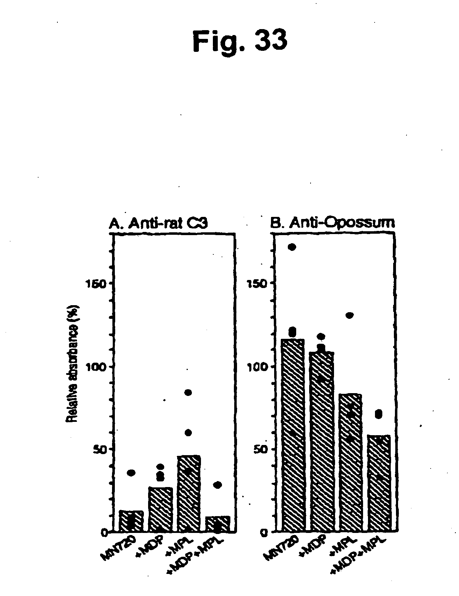

Comparative ELISA analyses were performed on sera from week 5 of the treatment program. Absorbances were measured, and the values were compared to a relative absorbance with Freund's set at 100%. The addition of MLP, MPL, fMLP, or a combination of two or three of these did not have any significant positive effect on the response against either the self nor the non-self epitopes when administrated together with MN51 (Figure 32A and B). A slight negative effect on the anti-self-response was observed with several of these additions (Figure 32A). In the experiment with MN720, a minor enhancement of the anti-self-response was seen with MPL (Figure 33A). All additions had a minor negative effect on the anti non-self response, however (Figure 33B).

### Example 4 - An analysis of ORO with alum

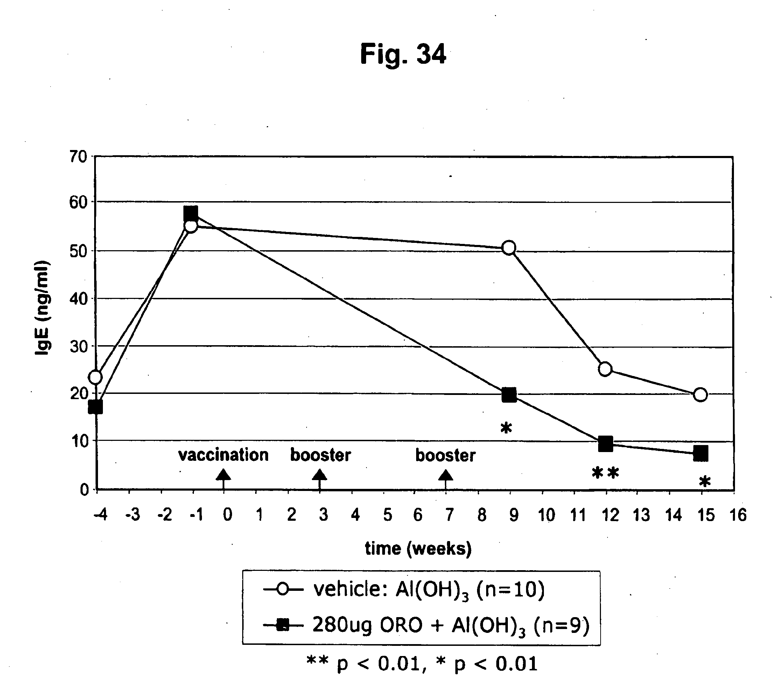

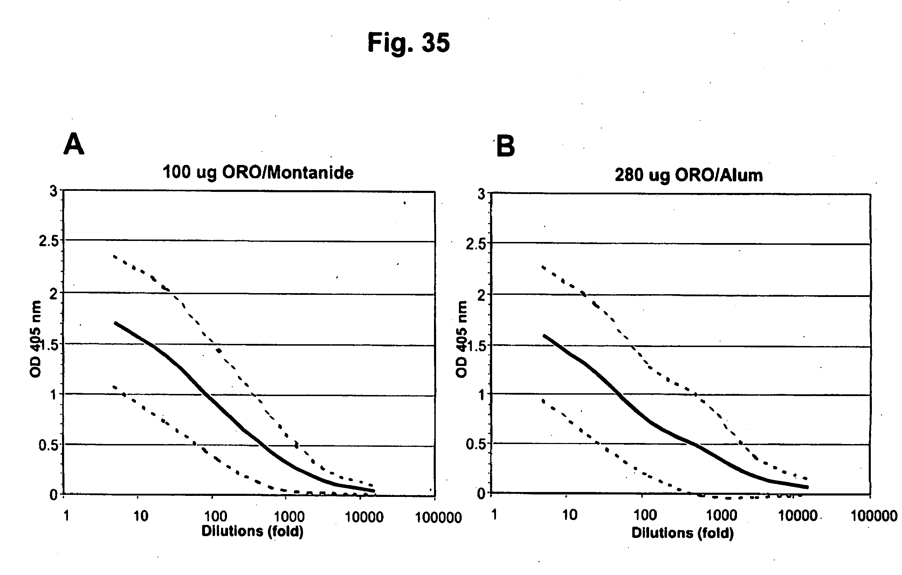

The effectiveness of alum was further tested in another study, in which the alum was prepared from Rehydragel LG. After four weeks of sensitization with OVA, groups of 9 or 10 female Wistar rats were subcutaneously immunized with compositions containing vehicle (PBS) with alum, 100 µg H-ORO with MN51, or 280 µg H-ORO with alum. Booster immunizations were given at weeks 3 and 7. Serum samples were obtained at weeks -4, -1, 9, 12, and 15. The samples from week 12 were analyzed for titer dilution, while all samples were analyzed for free IgE concentrations using standard methods. The concentration of free IgE diminished over time in the sera of animals injected with either vehicle plus alum or H-ORO plus alum (Figure 34). However, the decrease was greater in the animals treated with H-ORO plus alum, and the concentrations of free IgE after 9 weeks were significantly different between the two groups (p < 0.01). Immunization with 280 µg H-ORO and alum resulted in a dilution curve that was very similar to that displayed by sera from animals immunized with 100 µg H-ORO and MN51 (Figure 35A and 35B). In fact, the titer dilution₅₀ values were calculated to be 400-fold for ORO with alum and 204-fold for H-ORO with MN51. These results demonstrate that a composition containing greater than 100 µg H-ORO in combination with alum can induce substantial anti-self IgE responses when administered to a mammal.

### Example 5 - Analysis of ORO-H and ORORO-H

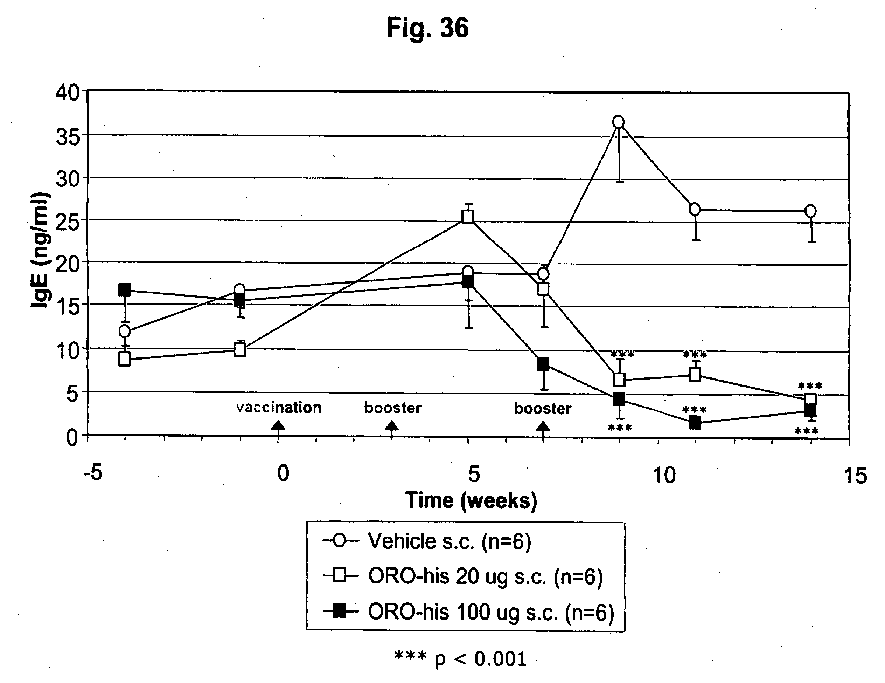

Similar studies were conducted to evaluate the effects of compositions containing ORO and ORORO linked to a His tag. The ORORO-H polypeptide contains the following IgE domains: OpossumCH2-RatCH3-OpossumCH2-RatCH3-OpossumCH4. After four weeks of sensitization with OVA, groups of 6 male Wistar F rats were subcutaneously immunized with compositions containing vehicle (PBS), 20 µg ORO-H, or 100 µg ORO-H. Booster immunizations were given at weeks 3 and 7. In each case, Montanide ISA 51 was used as an adjuvant. Serum samples were obtained at weeks -4, -1, 5, 7, 9, 11, and 14, and were analyzed for free IgE concentrations. As shown in Figure 36, immunization with either 20 µg ORO-H or 100 µg ORO-H was equally effective at reducing the concentration of free IgE, while the vehicle resulted in an increase in free IgE levels.

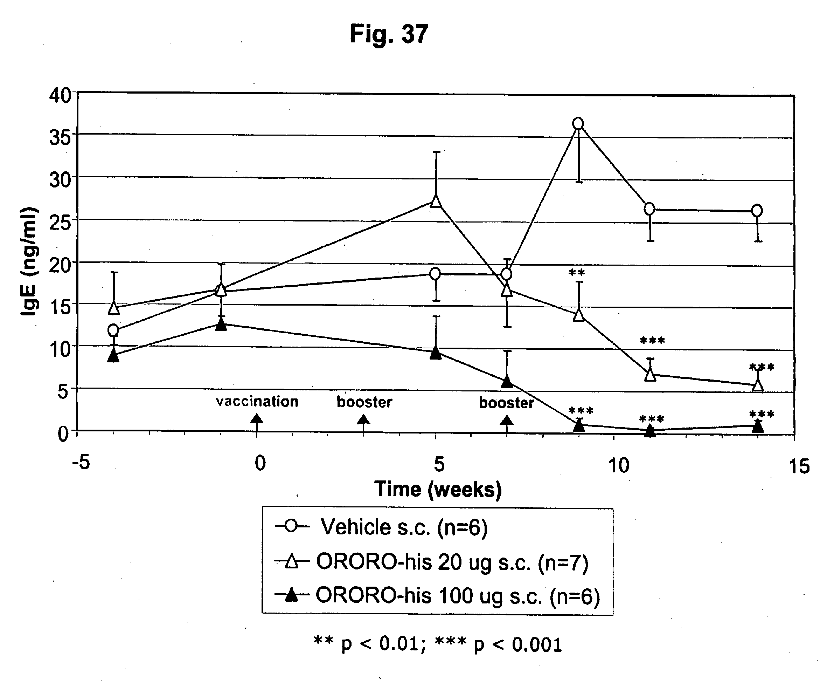

In another experiment, groups of 6 or 7 male Wistar rats received either PBS vehicle, 20 µg ORORO-H, or 100 µg ORORO-H via subcutaneous injection. Booster immunizations were given at weeks 3 and 7, and serum samples were obtained at weeks -4, -1, 5, 7, 9, 11, and 14. In each case, Montanide ISA 51 was used as an adjuvant. As in the experiment described in the paragraph immediately above, immunization with either 20 or 100 µg of ORORO-H was highly effective at reducing the concentration of free IgE, while the vehicle resulted in an increase in free IgE levels (Figure 37).

### Example 6 - Dose and toxicity studies in rat

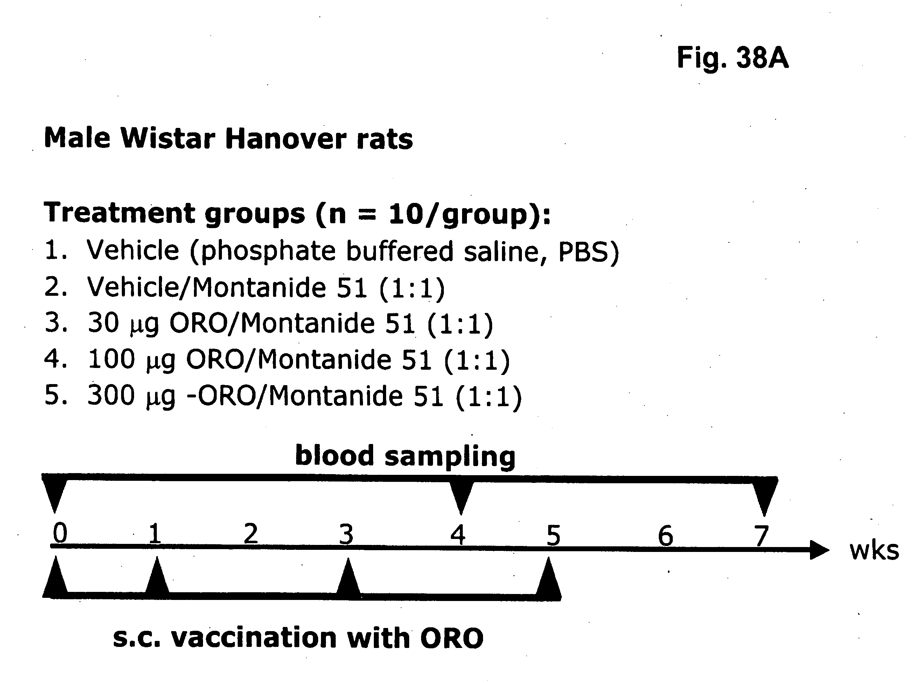

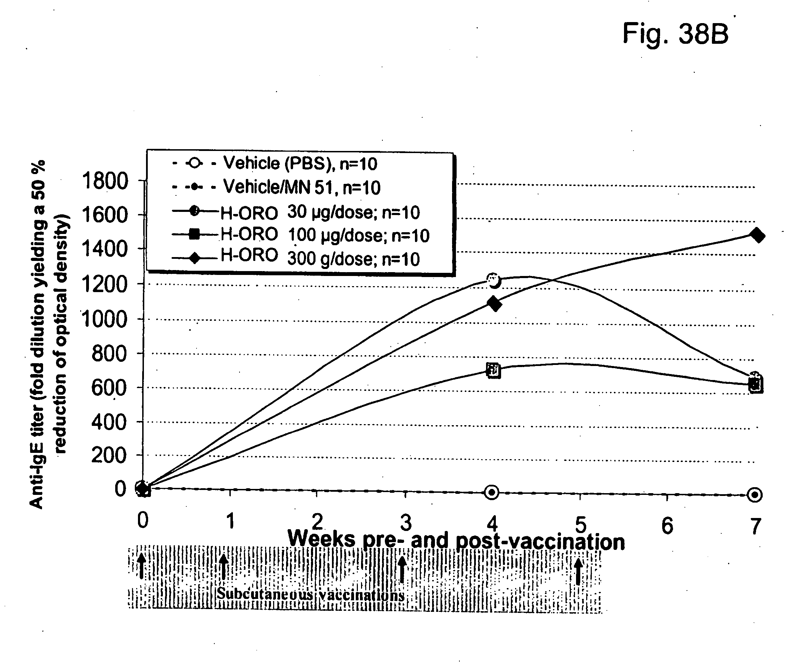

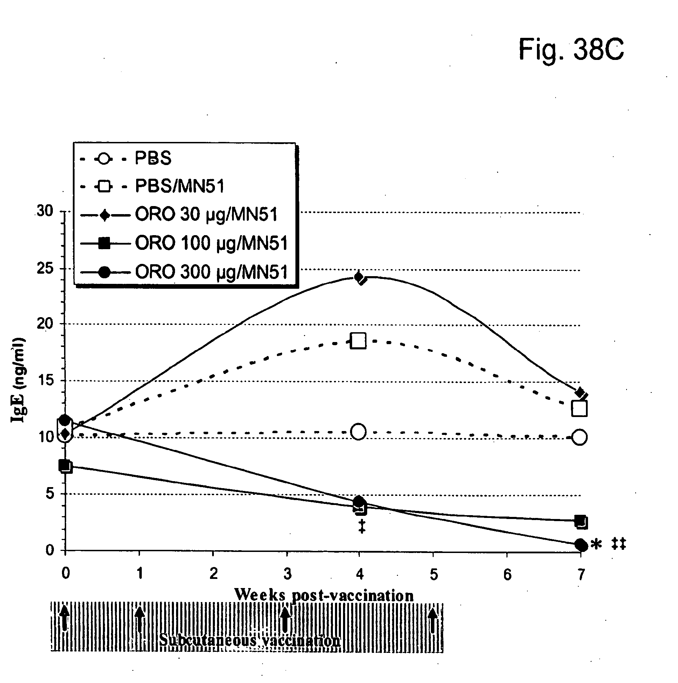

Groups of 10 male Wister Hanover rats were subcutaneously immunized with vehicle (PBS), vehicle with MN51, 30 µg H-ORO with MN51, 100 µg H-ORO with MN51, or 300 µg H-ORO with MN51. All mixtures with MN51 were in a 1:1 ratio. Booster immunizations were given at weeks 1, 3, and 5, and blood samples were obtained at weeks 0, 4, and 7 (Figure 38A). The median titer dilution₅₀ values at week 7 increased as the dose of H-ORO increased (Figure 38B). Thus, higher doses of H-ORO administered with MN51 can result in a greater anti-self IgE effect. In addition, free IgE antibody levels were reduced in animals receiving either 100 or 300 µg of H-ORO in MN51 (Figure 38C).

Toxicity and general health studies also were conducted using these animals. Blood samples were evaluated for albumin, ASAT and ALAT, bilirubin, creatinine, electrolytes such as Ca²⁺, K⁺, and Na⁺, lactate dehydrogenase, γ-glutamyl transpeptidase, and glucose, as well as haemoglobin, white blood cells, hematocrit, and platelet count. In addition, the animals were monitored twice weekly for body weight, once daily for changes in food intake, and for general physical activity, behavior, and appearance. Furthermore, histopathology studies were conducted on brain, lungs, ileum, liver, heart, spleen, kidneys, and testicles. In all of these examinations, no signs of toxic or unwanted effects were observed.

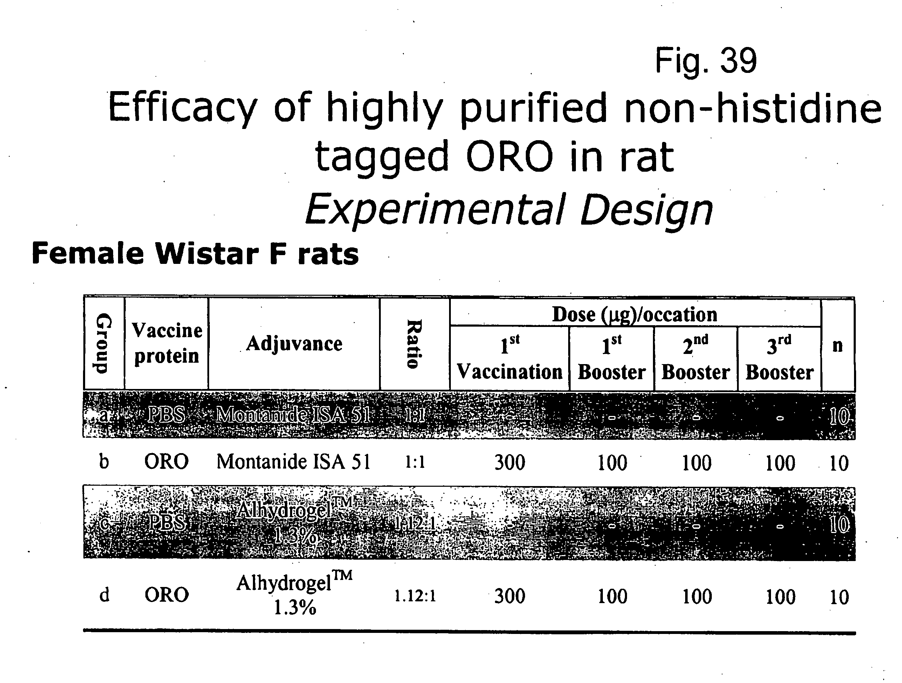

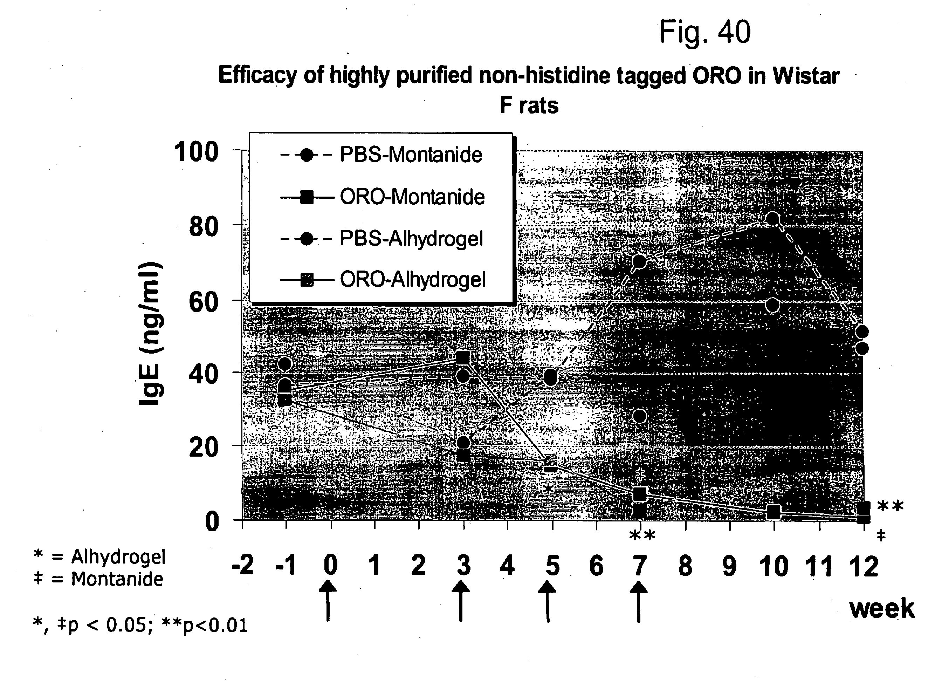

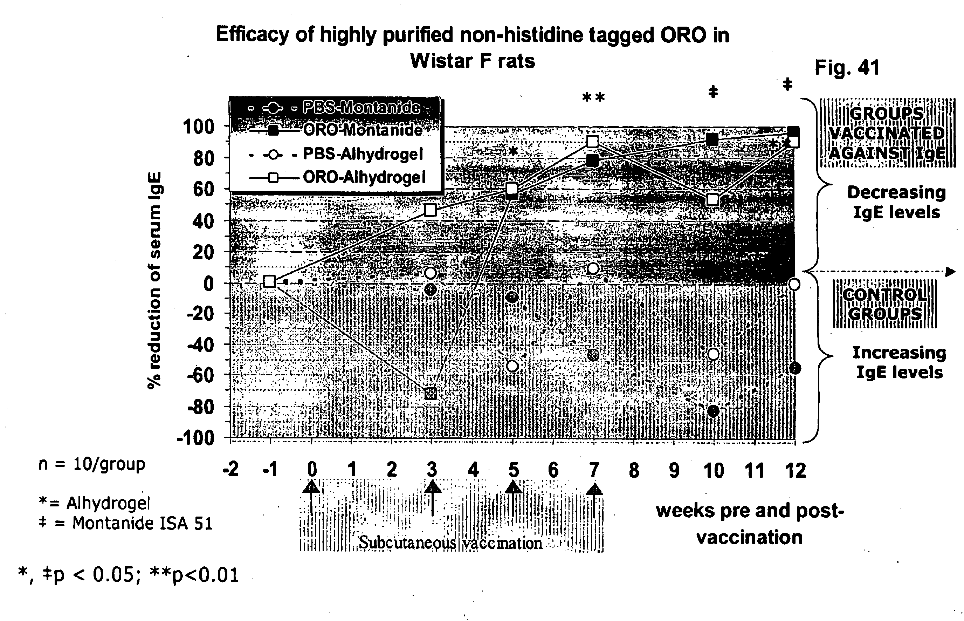

In another experiment, groups of 10 female Wister F rats were subcutaneously immunized with vehicle (PBS) or ORO lacking a histidine tag (Figure 39). The adjuvant was either MN51 or Alhydrogel^{™} 1.3% (an aluminum hydroxide gel adjuvant; Brenntag Stinnes Logistics). Booster immunizations were given at weeks 3, 5, and 7, and blood samples were obtained at weeks -1, 3, 5, 7, 10, and 12. The amount of rat IgE antibodies measured in rats receiving the ORO polypeptide were significantly reduced as compared to the amounts measured in control rats (Figure 40). In fact, rats receiving the ORO polypeptide with alum or MN51 exhibited 90-100 percent reductions in the amount of free rat IgE (Figure 41). These results demonstrate that chimeric IgE polypeptides in combination with alum or MN51 can reduce the levels of free IgE present within a mammal.

### Example 7 - Studies in cynomolgus monkeys

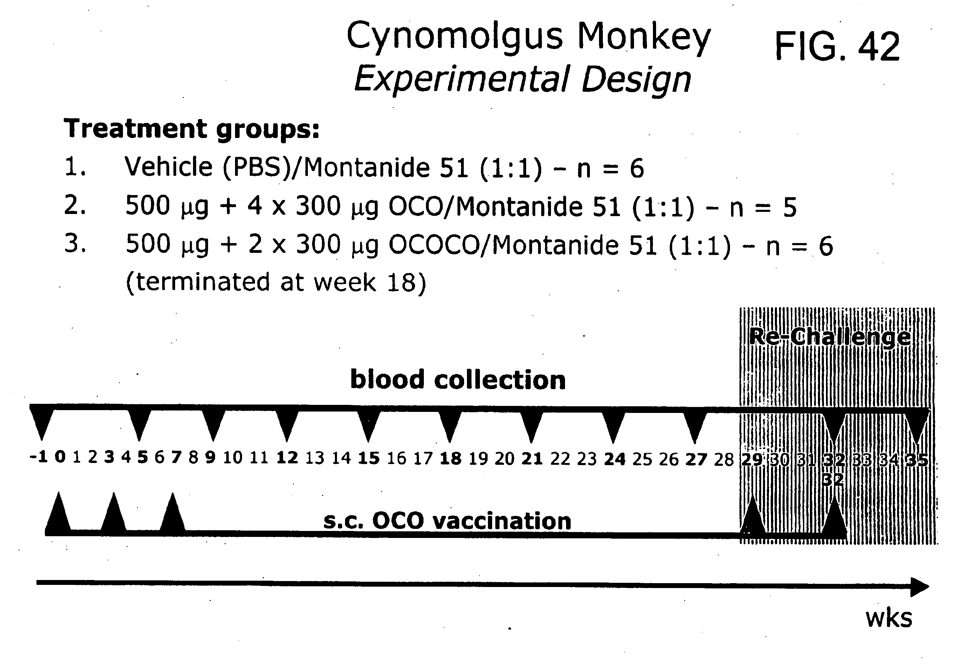

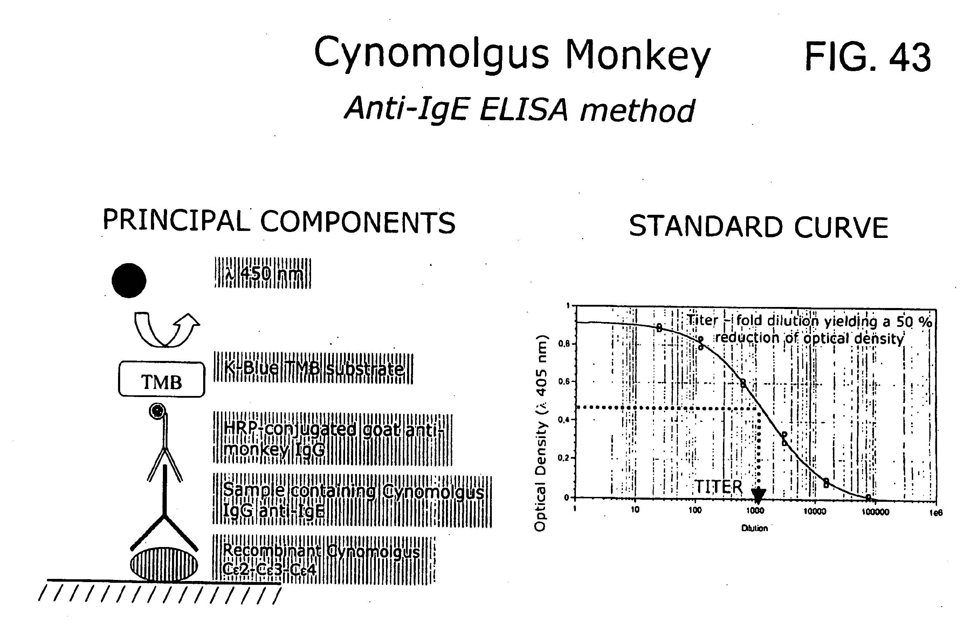

The ability of the compositions provided herein to elicit an anti-self IgE antibody response also was examined in cynomolgus monkeys. H-OCO-H and H-OCOCO-H polypeptides were prepared that were similar to the ORO and ORORO polypeptides described herein, with the exception that the rat IgE segments were replaced with IgE segments from cynomolgus monkey. Groups of 5 or 6 animals were subcutaneously immunized with vehicle (PBS) plus MN51, 500 µg H-OCO-H plus MN51, or 500 µg H-OCOCO-H plus MN51 (Figure 42). Booster immunizations (300 µg) were given at weeks 3 and 7, while re-boosters (300 µg) were given at weeks 29 and 32. Blood samples were obtained at weeks -1, 5, 9, 12, 15, 18, 21, 24, 27, 32 and 35. The anti-IgE responses were measured against a recombinant part of the constant domain (C∈2-C∈3-C∈4) of cynomolgus monkey IgE (Figure 43). Titer dilution₅₀ values were measured for each week that samples were obtained.

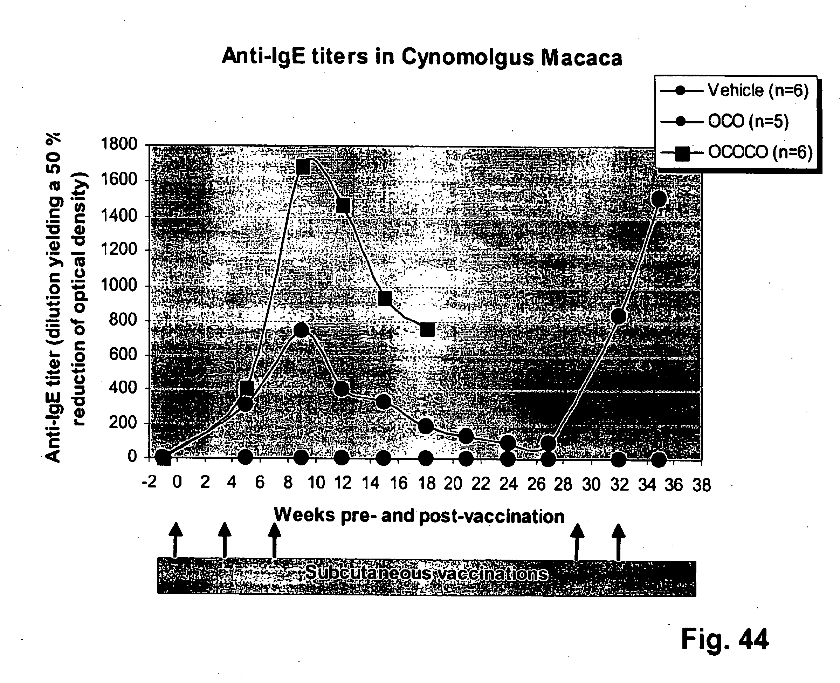

Immunization with either H-OCO-H or H-OCOCO-H resulted in an increase in titer dilution₅₀ that reached a maximum level at 9 or 10 weeks and then decreased (Figure 44). H-OCOCO-H induced a slightly greater effect than H-OCO-H, although the difference was not significant (Figure 44). The study using the H-OCOCO-H group was terminated at week 18.

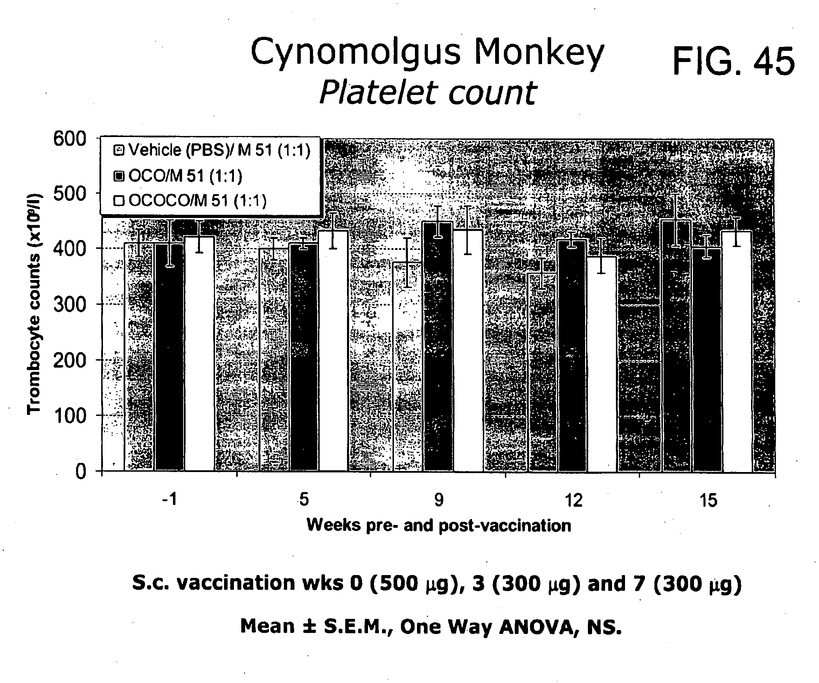

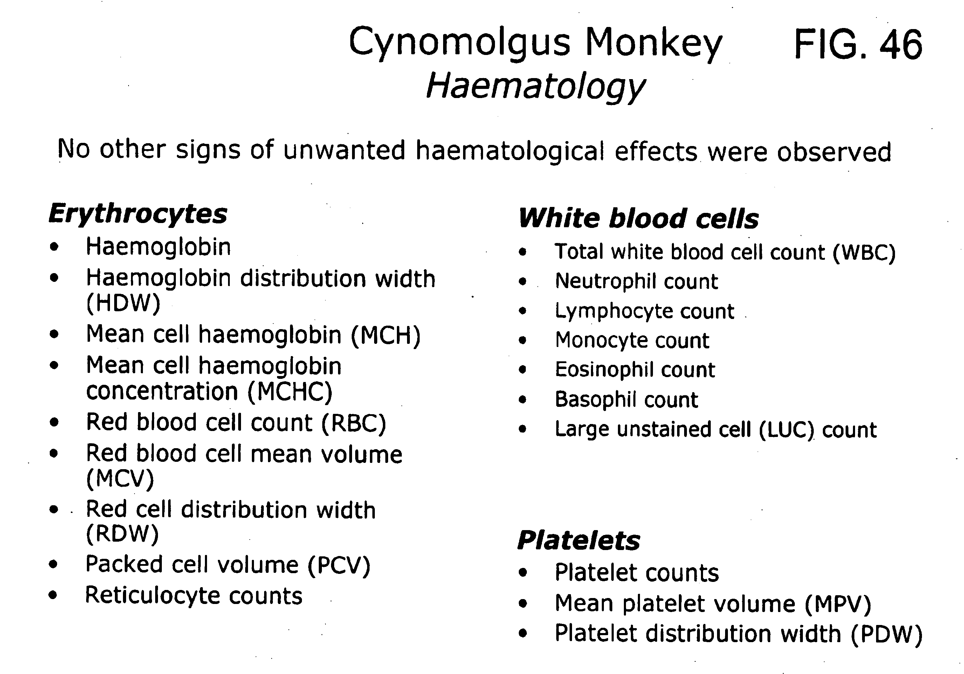

The anti-IgE response to H-OCO-H decreased over time, demonstrating that the effect is reversible (Figure 44). To determine whether the anti-IgE response is repeatable, the previously vaccinated animals were challenged with H-OCO-H at weeks 29 and 32. Animals previously exhibiting an anti-IgE response exhibited a second anti-IgE response (Figure 44). The H-OCO-H and H-OCOCO-H vaccines did not produce unwanted haematological effects on thrombocyte counts (Figure 45) or other blood cells (Figure 46). This experiment demonstrated that compositions containing a polypeptide such as H-OCO-H or H-OCOCO-H can be used in combination with MN51 to stimulate an anti-self IgE antibody response in primates, and that similar compositions could be developed for use in humans.

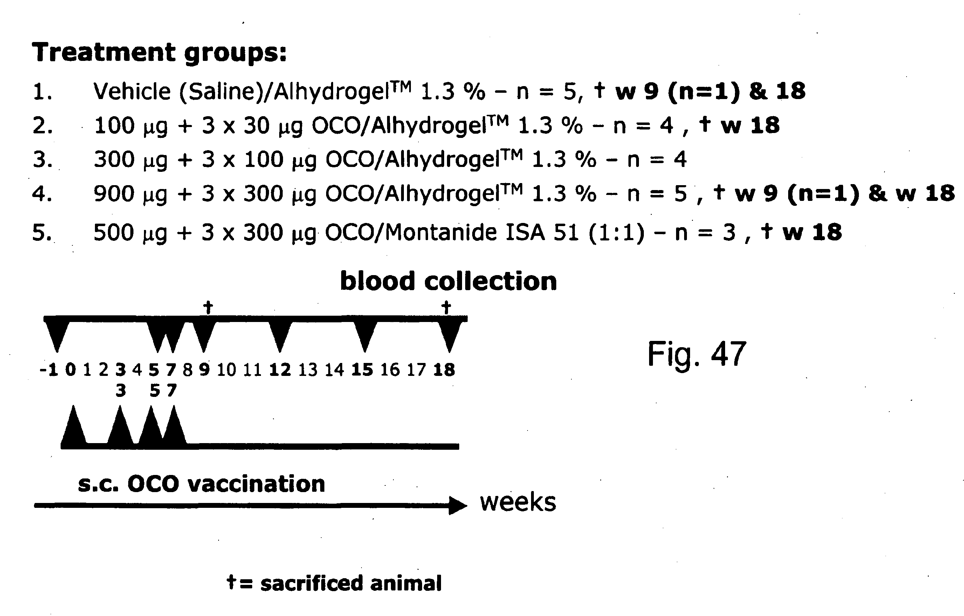

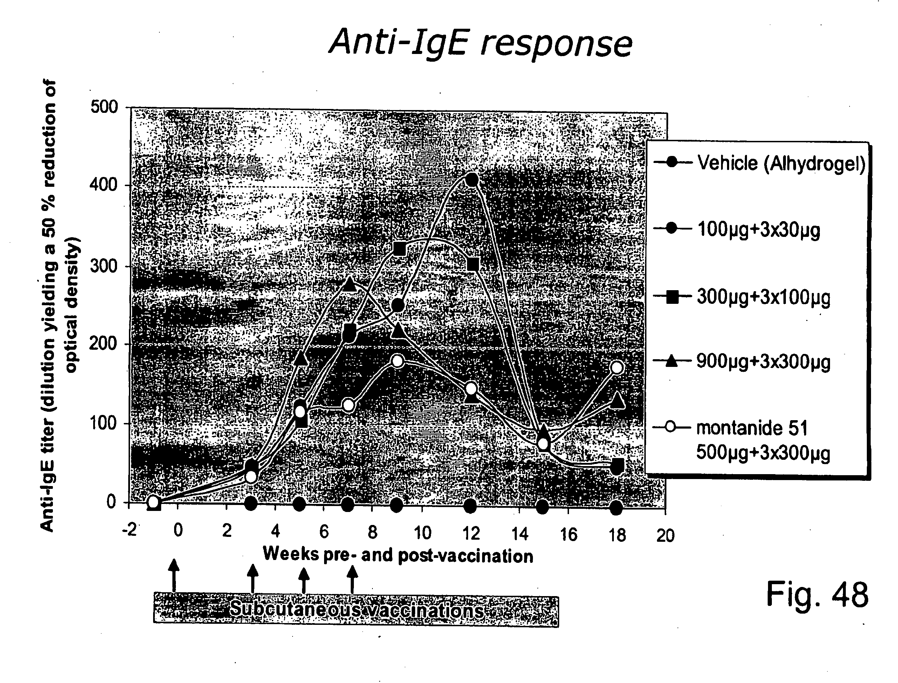

In another experiment, groups of cynomolgus monkeys were subcutaneously immunized with H-OCO-H in combination with either Alhydrogel^{™} 1.3% (an aluminum hydroxide gel adjuvant; Brenntag Stinnes Logistics) or MN51 (Figure 47). Control monkeys were immunized with saline in combination with Alhydrogel^{™} 1.3%. Booster immunizations were given at weeks 3, 5, and 7, and blood samples were obtained at weeks -1, 3, 5, 7, 9, 12, 15, and 18. The amount of monkey anti-IgE antibodies measured in monkeys receiving the H-OCO-H polypeptide were significantly increased as compared to the amounts measured in control monkeys (Figure 48). In addition, monkeys receiving the H-OCO-H polypeptide in combination with alum produced a stronger anti-IgE antibody response than the response produced by monkeys treated with the H-OCO-H polypeptide in combination with MN51 (Figure 48).

These results demonstrate that chimeric IgE polypeptides with alum or MN51 can break a primate's self tolerance to IgE. These results also demonstrate that the anti-IgE responses are reversible and repeatable. In addition, primates treated with chimeric IgE polypeptides with alum or MN51 exhibited no signs of thrombocytopenia or other unwanted hematological effects.

## Claims

1. A composition comprising a polypeptide and an aluminum compound, wherein said polypeptide is a chimeric IgE polypeptide comprising a self IgE polypeptide sequence, and wherein administration of said composition to a mammal reduces the level of detectable free IgE in said mammal.

2. The composition of claim 1, wherein said polypeptide comprises a sequence set forth in SEQ ID NO : 3, SEQ ID NO : 6, SEQ ID NO : 10, SEQ ID NO : 12, SEQ ID NO : 14, SEQID NO : 16, SEQID NO : 18, or SEQID NO :21.

3. The composition of claim 1, wherein said composition comprises between about ten micrograms and about one gram of said polypeptide per unit dose.

4. The composition of claim 1, wherein said composition comprises about 280 micrograms of said polypeptide per unit dose.

5. The composition of claim 1, wherein said aluminum compound is an aluminum hydrogel compound.

6. The composition of claim 1, wherein said aluminum compound is alum.

7. The composition of claim 6, wherein said composition comprises between about ten microliters and about one milliliter of said alum per unit dose.

8. The composition of claim 6, wherein said composition comprises about 50 microliters of said alum per unit dose.

9. The composition of claim 1, wherein said reduction is at least about a 10 percent reduction.

10. The composition of claim 1, wherein said reduction is at least about a 30 percent reduction.

11. The composition of claim 1, wherein said reduction is a reduction from about 10 percent to about 95 percent.

12. The composition of claim 1, wherein said reduction is a reduction from about 20 percent to about 95 percent.

13. The composition of claim 1, wherein said reduction is detectable in an ELISA.

14. The composition of claim 13, wherein an IgE receptor polypeptide sequence is used in said ELISA.

15. The composition of claim 1, wherein said administration of said composition to said mammal produces an anti self IgE antibody response with a titer dilutionson₅₀ value greater than 100.

16. The composition of claim 15, wherein said titer dilution₅₀ value is greater than 200.

17. The composition of claim 15, wherein said titer dilution₅₀ value is greater than 400.

## Patentansprüche

1. Zusammensetzung, umfassend ein Polypeptid und eine Aluminiumverbindung, wobei das Polypeptid ein chimäres IgE-Polypeptid mit einer eigen IgE-Polypeptidsequenz ist, und wobei die Verabreichung der Zusammensetzung an ein Säugetier den Level an detektierbarem freien IgE bei diesem Säugetier senkt.

2. Zusammensetzung nach Anspruch 1, wobei das Polypeptid eine Sequenz, wie gezeigt in SEQ ID Nr. 3, SEQ ID Nr. 6, SEQ ID Nr. 10, SEQ ID Nr. 12, SEQ ID Nr. 14, SEQ ID Nr. 16, SEQ ID Nr. 18 oder SEQ ID Nr. 21, umfasst.

3. Zusammensetzung nach Anspruch 1, wobei die Zusammensetzung zwischen ungefähr zehn Mikrogramm und ungefähr ein Gramm des Polypeptids pro Einheitsdosis umfasst.

4. Zusammensetzung nach Anspruch 1, wobei die Zusammensetzung ungefähr 280 Mikrogramm des Polypeptids pro Einheitsdosis umfasst.

5. Zusammensetzung nach Anspruch 1, wobei die Aluminiumverbindung eine Aluminium-Hydrogel-Verbindung ist.

6. Zusammensetzung nach Anspruch 1, wobei die Aluminiumverbindung Alaun ist.

7. Zusammensetzung nach Anspruch 6, wobei die Zusammensetzung zwischen ungefähr zehn Mikrolitern und ungefähr einen Milliliter des Alauns pro Einheitsdosis umfasst.

8. Zusammensetzung nach Anspruch 6, wobei die Zusammensetzung ungefähr 50 Mikroliter des Alauns pro Einheitsdosis umfasst.

9. Zusammensetzung nach Anspruch 1, wobei die Senkung zumindest ungefähr eine 10%-Senkung ist.

10. Zusammensetzung nach Anspruch 1, wobei die Senkung zumindest ungefähr eine 30%-Senkung ist.

11. Zusammensetzung nach Anspruch 1, wobei die Senkung eine Senkung von ungefähr 10 Prozent bis ungefähr 95 Prozent ist.

12. Zusammensetzung nach Anspruch 1, wobei die Senkung eine Senkung von ungefähr 20 Prozent bis ungefähr 95 Prozent ist.

13. Zusammensetzung nach Anspruch 1, wobei die Senkung in einem ELISA detektierbar ist.

14. Zusammensetzung nach Anspruch 13, wobei eine IgE-Rezeptor-Polypeptidsequenz im ELISA verwendet wird.

15. Zusammensetzung nach Anspruch 1, wobei die Verabreichung der Zusammensetzung an das Säugetier eine anti-eigen IgE-Antikörperantwort mit einem Titer-Dilutions₅₀-Wert größer als 100 produziert.

16. Zusammensetzung nach Anspruch 15, wobei der Titer-Dilutions₅₀-Wert größer als 200 ist.

17. Zusammensetzung nach Anspruch 15, wobei der Titer-Dilutions₅₀-Wert größer als 400 ist.

## Revendications

1. Composition comprenant un polypeptide et un composé d'aluminium, dans laquelle ledit polypeptide est un polypeptide chimère IgE comprenant une séquence de polypeptides auto-IgE, et dans laquelle une administration de ladite composition à un mammifère réduit le niveau d'IgE libre détectable dans ledit mammifère.

2. Composition selon la revendication 1, dans laquelle ledit polypeptide comprend une séquence présentée dans SEQ ID No. : 3, SEQ ID No. : 6, SEQ ID No. : 10, SEQ ID No. : 12, SEQ ID No. : 14. SEQ ID No. : 16, SEQ ID No. : 18, SEQ ID No. : 21.

3. Composition selon la revendication 1, dans laquelle ladite composition comprend entre environ dix microgrammes et environ un gramme dudit polypeptide par dose unitaire.

4. Composition selon la revendication 1, dans laquelle ladite composition comprend environ 280 microgrammes dudit polypeptide par dose unitaire.

5. Composition selon la revendication 1, dans laquelle ledit composé d'aluminium est un composé hydrogel d'aluminium.

6. Composition selon la revendication 1, dans laquelle ledit composé d'aluminium est de l'alun.

7. Composition selon la revendication 6, dans laquelle ladite composition comprend entre environ dix microlitres et environ un millilitre dudit alun par dose unitaire.

8. Composition selon la revendication 6, dans laquelle ladite composition comprend environ 50 microlitres dudit alun par dose unitaire.

9. Composition selon la revendication 1, dans laquelle ladite réduction est au moins environ une réduction de 10 pour cent.

10. Composition selon la revendication 1, dans laquelle ladite réduction est au moins environ une réduction de 30 pour cent.

11. Composition selon la revendication 1, dans laquelle ladite réduction est une réduction d'environ 10 pour cent à environ 95 pour cent.

12. Composition selon la revendication 1, dans laquelle ladite réduction est une réduction d'environ 20 pour cent à environ 95 pour cent.

13. Composition selon la revendication 1, dans laquelle ladite réduction est détectable dans un ELISA.

14. Composition selon la revendication 13, dans laquelle une séquence de polypeptides de récepteur d'IgE est utilisée dans ledit ELISA.

15. Composition selon la revendication 1, dans laquelle ladite administration de ladite composition audit mammifère produit une réponse d'anticorps anti auto-IgE avec une valeur de dilution₅₀ de titre supérieure à 100.

16. Composition selon la revendication 15, dans laquelle ladite valeur de dilution₅₀ de titre est supérieure à 200.

17. Composition selon la revendication 15, dans laquelle ladite valeur de dilution₅₀ de titre est supérieure à 400.
